# EUROPEAN PATENT SPECIFICATION

(11) **EP 1 008 594 B1**
(45) Date of publication and mention of the grant of the patent: **17.03.2004**
(21) Application number: 98930807.7
(22) Date of filing: 01.07.1998
(51) Int. Cl.: C07D 333/22, C07D 333/56, C07D 333/58, C07D 333/42, A61K 31/495

(54) **COMPOUNDS DERIVED FROM THOPHENE AND BENZOTHIOPHENE, AND RELATED UTILISATION AND COMPOSITION**
THIOPHEN- UND BENZOTHIOPHENDERIVATE, DEREN VERWENDUNG UND SIE ENTHALTENDE ZUBEREITUNG
COMPOSES DERIVES DE THIOPHENE ET DE BENZOTHIOPHENE, LEUR UTILISATION ET COMPOSITION

(30) Priority: 08.07.1997 ES 9701517
(43) Date of publication of application: 14.06.2000
(73) Proprietor: LABORATORIOS VITA, S.A., 08970 Sant Joan Despi (Barcelona) (ES)
(72) Inventor: MONGE VEGA, Antonio, E-31190 Cizur-Menor (ES); DEL RIO ZAMBRANA, Joaquin, E-28036 Madrid (ES); LASHERAS ALDAZ, Berta, E-Baranain (ES); PALOP CUBILLO, Juan Antonio, E-31008 Pamplona (ES); BOSCH ROVIRA, Anna, E-08017 Barcelona (ES); DEL CASTILLO NIETO, Juan Carlos, E-08027 Barcelona (ES); ROCA ACIN, Juan, E-08970 Sant Joan Despi (ES)
(74) Representative: Curell Aguilà, Marcelino
(86) International application number: PCT/ES1998/000191
(87) International publication number: WO 1999/002516

(56) References cited:
- EP-A- 0 574 313
- EP-A- 0 596 120
- GB-A- 1 096 341
- US-A- 2 979 507
- US-A- 3 002 976
- US-A- 4 515 793

## Description

### Field of the Invention

The present invention relates to benzothiophene derivative compounds, the salts, optical isomers and polymorphs thereof, having the general formula (I) 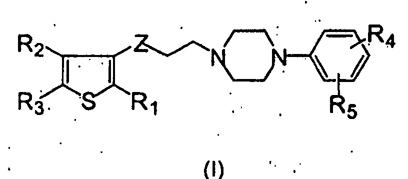 R₂ and R₃ together are: -C(R₈)= C(R₉)- C(R₁₀)= C(R₁₁)- and wherein R₈, R₉, R₁₀ and R₁₁ are independent and stand for H, C₁-C₆ alkyl, halogen, -OR₁₂, nitro, cyano, NR₁₃R₁₄; COR₁₂,CO₂R₁₂; SO₂NR₁₃R₁₄; -SO₂R₁₂; SR₁₂, -CONR₁₃R₁₄; wherein R₁₂-R₁₄ are as defined herein after and also relates to the corresponding pharmaceutical compositions and the use thereof in the preparation of these pharmaceutical compositions for the treatment of neurological disorders and, in particular, for the treatment of anxiety and/or depression, as being antidepressants having a dual activity, 5-HT_{1A} antagonism and inhibition of serotonin reuptake.

### Background of the Invention

Drugs for the treatment of depression have been available for over 30 years. Both the first monoamine oxidase inhibitor (MAO inhibitor), iproniazide, and the first tricyclic antidepressant (TCA), imipramine, were placed on the market at the end of the 50's. The second-generation antidepressants represent a considerable improvement on the traditional tricyclic antidepressants, or on the irreversible unspecific MAO inhibitors. In spite of this, they still offer side effects, and what is more important, the latency time until the therapeutic effect appears is still too long for the treatment to be deemed optimal.

The latest class of antidepressants placed on the market was the one comprising the selective serotonin reuptake inhibitors, outstanding among which are fluoxetine (Lilly ES433720), paroxetine (Ferrosan, ES422734) and sertraline (Pfizer, ES496443). The products of this class have a high degree of structural diversity in comparison with other types of serotonin reuptake inhibitors, such as may be the tricyclic antidepressants. In spite of their structural variety, these compounds are highly selective for the serotonin receptor. In fact, their binding to α and β adrenergic, dopaminergic, histamine and muscarine receptors is insignificant. It is postulated that this could be due to a great structural similarity to the pharmacophore, which is responsible for their specificity, and relative affinity to the corresponding serotonin receptor.

Among the most frequent adverse effects of the serotonin reuptake inhibitors are those related with gastrointestinal disorders. The majority of them also cause inhibition of the hepatic metabolism of other drugs with the corresponding pharmaco-dynamic interactions and have a retarded onset of their antidepressant action.

With this background in mind, there arises the need to continue investigating so as to create a third generation of antidepressants. The four points that an antidepressant must fulfil to be considered as a member of the third generation are:
- 1.: Faster action
- 2.: Broader efficacy
- 3.: Less side effects
- 4.: Safer in case of overdose

The first of these four points is the one offering the greatest challenge in antidepressant research, since the harm that it represents for a depression patient that the drug does not start to show its effects until the elapse of several weeks after the start of the treatment is obvious.

The reason why the ailment takes time to remit, after treatment with monoamine reuptake inhibitors, appears to be due to a process of desensitisation of the presynaptic 5-HT_{1A} receptors, which means that the serotoninergic tone is reduced until this desensitisation has occurred.

It may be gathered from all the above that an antidepressant treatment which, further to inhibiting the serotonin reuptake, were to involve a blocking of the 5-HT_{1A} somatodentritic autoreceptors would increase the antidepressant effectiveness, on allowing the serotonin concentration in the serotoninergic terminations to rise from the very onset. In this sense, there has been proposed the simultaneous administration of serotonin reuptake inhibitors with selective 5-HT_{1A} receptor antagonists, such as pindolol (Artigas F. et al., Arch. Gen. Psychiatry, **51**, 248-251 (1994); Blier P. et al., J. Clin. Pharmacol. **15**, 217-222 (1995)) to facilitate the quickest possible onset of the antidepressant effect. This theory has led the researchers suggest that the addition of products blocking the 5-HT_{1A} type autoreceptors may prevent the onset of this negative feedback system and potentiate the effect of the serotonin reuptake inhibitors.

One Lilly patent (EP 0 687 472) claims the potentiation of the effect of the serotonin reuptake inhibitors by increasing the availability of certain brain neurotransmitters (serotonin among them) by combining the serotonin reuptake inhibitors with selective 5-HT_{1A} receptor antagonists. Among the 5-HT_{1A} antagonists of greater interest cited in said patent, one should mention prenalol, WAY 100135, spiperone, pindolol, (S)-UH-301, penbutolol, propranolol and tetratolol among others. There may also be added to this group of products a highly selective specific 5-HT_{1A} inhibitor, WAY-100635 (Fornal C.A. et al., Brit. J. Pharmacol., **112**, (2), 92P (1994); Fletcher A. et al., Brit. J. Pharmacol. **112**, (2) 91P (1994))

Bearing the above background in mind, it is therefore an object of this invention to synthesise compounds having this dual activity, i.e., serotonin reuptake inhibitors with antagonistic activity towards the 5-HT_{1A} receptor.

The present invention relates in particular to the synthesis and pharmacological activity of new benzothiophene derivatives of the general formula (I).

Some products similar to those disclosed here have been claimed in the literature. Thus, for exampte, US patent 2,979,507 claims products having the general formula:

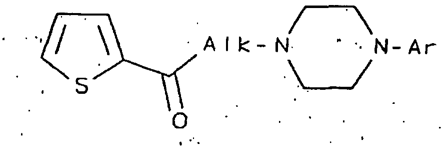

To be precise, there are disclosed the products: 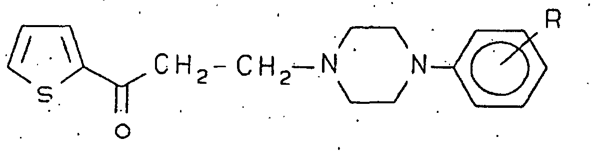 where R may be H or 2-OCH₃ among others.

The document EP 0 596 120 claims products of the general formula: 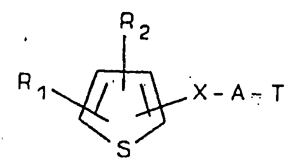 where X is generally: -S, or S(O)- but may be, among others: -C(O)-; -CH(OR)-; -C(N-OR)- ; -CH(NH₂)-; A may be an alkylene group and T is generally a 1,2-benzisoxazole or 1,2-benzothiazole ring, but may be any other aromatic ring. Nevertheless, the above document does not describe any of the products claimed in the present invention and, on the other hand, the products of said document are claimed as antipsychotics but not as antidepressants.

The document GB 1096341 discloses products of the general formula: 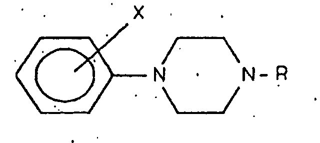 where R may be: -CH₂-CH₂-C(O)-Ar and Ar may be, among others, a thiophene ring.

To be precise, the above patent describes the products: 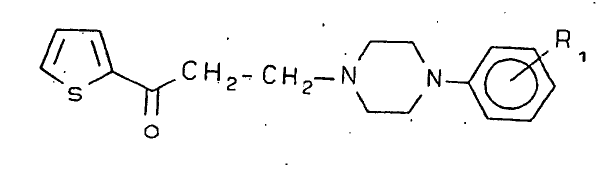 where R₁ is 2-F or 4-F or 4-Cl, which substituents will be excluded from the present invention by means of a disclaimer. In any case, no therapeutic action as antidepressants is claimed for the products disclosed in the said document GB 1096341.

US patent 3,002,976 claims compounds of the general formula: 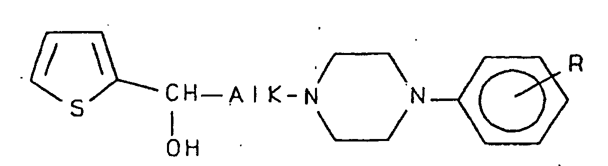 where R is H, methyl or halogen. These products are not comprised either under the present invention.

The document US 4,515,793 claims products similar to those of the present invention where the aromatic ring in the 4- position of the piperazine is always: 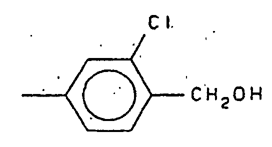 Products of this type are not the object of the present invention.

Documents US 2985657, US 2958694, US 2973360 and US 2975182 claim products similar to those of the present invention where the aromatic ring in the 4- position of the piperazine is a heterocycle (pyridine, pyrimidine, etc). Compounds of this type are not the object of the present invention.

Finally, documents US 2997472, BE 589092 and GB 1294720 describe products similar to those described in the present invention, but where the bond between the Z group (referred to in the present invention) and the piperazine ring is through an alkenyl chain having at least 3 carbon atoms.

### Description of the Invention

As stated above, the object of the present invention are the new benzothiophene derivatives of the general formula (I), and the corresponding compositions and the use thereof for obtaining compositions having a pharmacological activity. 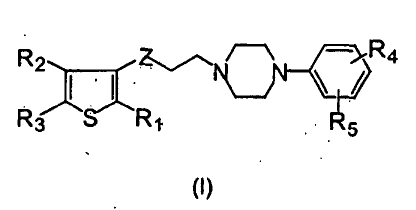 where
Z is: -CO-, -CH(OR₆)-, -C(NOR₇)-;
R₁ is: H, C₁-C₆ alkyl, halogen, or -OR₆;
R₂ and R₃ together form, together with the carbon atoms to which they are attached, a six membered aromatic ring, which in turn may be substituted, i.e. R₂ and R₃ together are: -C(R₈) = C(R₉)- C(R₁₀) = C(R₁₁)-;
R₄ and R₅ are the same or different and stand for: H, C₁-C₆ alkyl, halogen, haloalkyl, -OR₁₂, nitro, NR₁₃R₁₄; -COR₁₂; CO₂R₁₂; -SO₂NR₁₃R₁₄; -SO₂R₁₂; SR₁₂, cyano; -CONR₁₃R₁₄ or R₄ and R₅ may form together a benzene ring fused to the phenyl ring;
R₆ is: H, C₁-C₆ alkyl, CO₂R₁₂, -C(O)NR₁₃R₁₄, naphthyl or phenyl optionally substituted by one or more substituents selected from among the following:
H, C₁-C₆ haloalkyl, C₁-C₆ alkyl, halogen, C₁-C₆ alkoxy, methylenedioxy, nitro, cyano;
R₇ is: H or C₁-C₆ alkyl;
R₈, R₉, R₁₀ and R₁₁ are independent and stand for: H, C₁-C₆ alkyl, halogen, -OR₁₂, nitro, cyano, NR₁₃R₁₄; -COR₁₂; -CO₂R₁₂; -SO₂NR₁₃R₁₄; -SO₂R₁₂; -SR₁₂, -CONR₁₃R₁₄;
R₁₂ is H, C₁-C₆ alkyl or phenyl; and R₁₃ and R₁₄ are independent and stand for: H, C₁-C₆ alkyl or phenyl or R₁₃ and R₁₄, together with the atom of N to which they are attached form a 5- or 6- membered ring in which there may optionally be an atom of N, O or S.

The invention also comprises the physiologically acceptable salts, solvates and salts of the solvates of the formula (I) compounds and which include the acid addition salts formed with inorganic and organic acids, for example hydrochlorides, hydrobromides, sulphates, nitrates, phosphates, formates, mesylates, citrates, benzoates, fumarates, maleates, lactates and succinates, among others. When a salt of a formula (I) compound is formed with a dicarboxylic 'acid, such as succinic acid, the salt may contain between one and two moles of the formula (I) compound per mole of acid.

The preferred salts are the hydrochlorides. The preferred solvates are the hydrates.

The formula (I) compounds also additionally comprise the geometric isomers CIS/TRANS (Z and E) when the Z group stands for: -C(NOR₇)- and the optical isomers (R and S) when Z stands for: -CH(OR₆)-, as well as the enantiomeric mixtures thereof.

The preferred compounds according to the invention are those of formula (I) where:
Z is: -C(O)-, -CH(OH)-, -CH(OR₆)- or -C(NOR₇)- attached to the 2- or 3-position of the thiophene ring;
R₁ is: H or lower alkyl;
R₄ is H or halogen;
R₅ is H, hydroxy or lower alkoxy;
R₆ is H or naphthyl;
R₇ is H;
R₈, R₉, R₁₀ and R₁₁ are independent and stand for: H, low molecular weight alkyl, halogen, OR₁₇, nitro, NR₁₃R₁₄;
R₁₂, R₁₃, R₁₄ are independent and stand for H or alkyl.

The compounds of the present invention are useful for the treatment of disorders related with the serotonin reuptake and other disorders related with the post- or presynaptic transmission of serotonin and in particular for the treatment of depression.

The treatments may be preventive or curative and are carried out by administration by any conventional way of a formula (I) compound or of a physiologically acceptable salt or solvate thereof.

More particularly, the present invention relates to the benzothiophene derivatives having the following chemical names:
1-(benzo[b]thiophen-3-yl)-3-[4-(2-methoxyphenyl)piperazin-1-yl]propan-1-ol
1-(benzo[b]thiophen-3-yl)-3-[4-(2-methoxyphenyl)piperazin-1-yl]propan-1-one
1-(benzo[b]thiophen-3-yl)-3-[4-(2-methoxyphenyl)piperazin-1-yl]propan-1-one oxime
1-(benzo[b]thiophen-3-yl)-3-[4-(2-hydroxyphenyl)piperazin-1-yl]propan-1-ol
1-(3,5-dimethylbenzo[b]thiophen-2-yl)-3-[4-(2-methoxyphenyl)piperazin-1-yl)propan-1-ol
1-(3,5-dimethylbenzo[b]thiophen-2-yl)-3-[4-(2-methoxyphenyl)piperazin-1-yl]propan-1-one
1-(3-methylbenzo[b]thiophen-2-yl)-3-[4-(2-hydroxyphenyl)piperazin-1-yl]propan-1-ol

In accordance therewith, the invention also provides an acceptable pharmaceutical composition for use in medicine, comprising: (a) a pharmaceutically effective amount of a formula (I) compound and/or a salt or solvate thereof, and (b) a pharmaceutically acceptable carrier for oral, sublingual, parenteral, retard or intranasal administration or in a form appropriate for administration by inhalation or insufflation.

The invention also relates to the use of a benzothiophene derivative of formula (I) for the preparation of a drug having therapeutical application as an antidepressant.

The pharmaceutical compositions for oral administration may be solid, as for example tablets or capsules prepared by conventional means with pharmaceutically acceptable carriers, or liquids such as for example aqueous or oil solutions, syrups, elixirs, emulsions or suspensions prepared by conventional means with pharmaceutically acceptable additives.

The formula (I) compounds and the physiologically acceptable salts or solvates thereof may be prepared by adaptation of the general methods related below.

### DESCRIPTION OF THE SYNTHESIS PROCESSES

Some examples of benzothiophenes and thiophenes are described below. Although the thiophenes are not object of the invention, they have been included on a comparative basis.

### Preparation of the ketones of formula (Ia)

The ketone derivatives of formula (Ia) (Z is C=O) 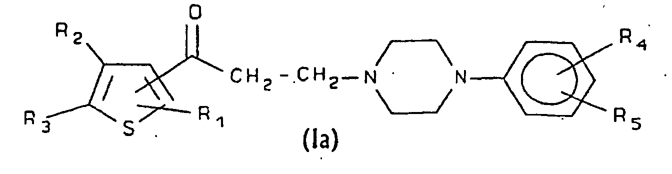 are prepared by:

### Method A

A Mannich reaction of the corresponding acylthiophene and acylbenzo(b)thiophene with the appropriate piperazine, according to the following reaction: 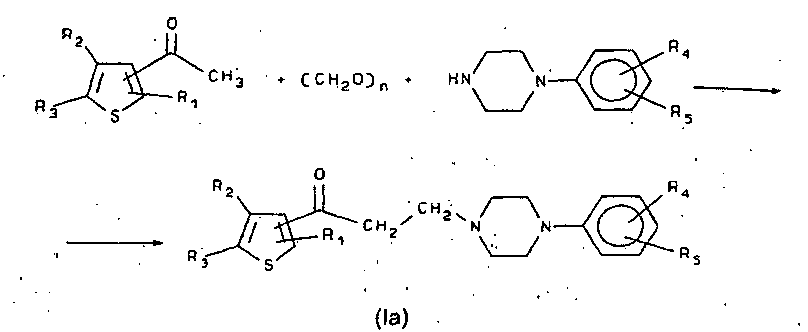 where R₁, R₂, R₃, R₄ and R₅ have the meanings given above.

### Method B

An alternative process for the preparation of the formula (la) ketones consists of reacting the corresponding 1-aryl-3-halo-1-propanone with the appropriate piperazine, according to the following reaction: 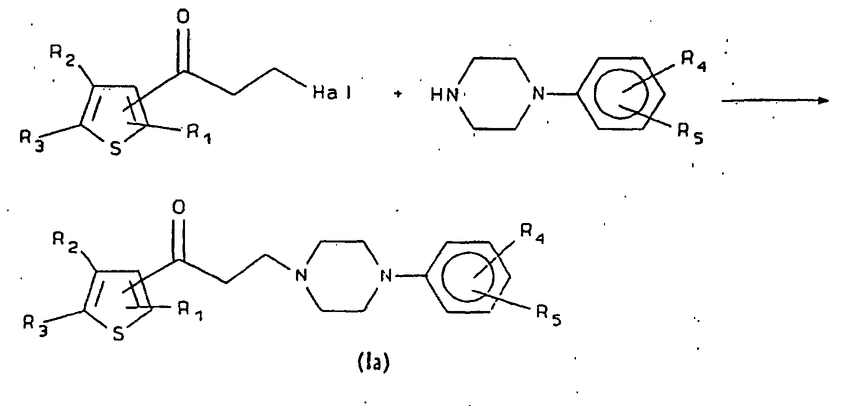 where Hal: halogen.

### Method C

A third process for the preparation of the formula (Ia) ketones consists of converting, by methods described in the literature, a substituent in a formula (Ia) compound into a different substituent, thereby obtaining another different compound responding structurally to the same type of formula (Ia). One example of such conversions consists of reducing an aromatic NO₂ group by methods described in the literature, to an amino group.

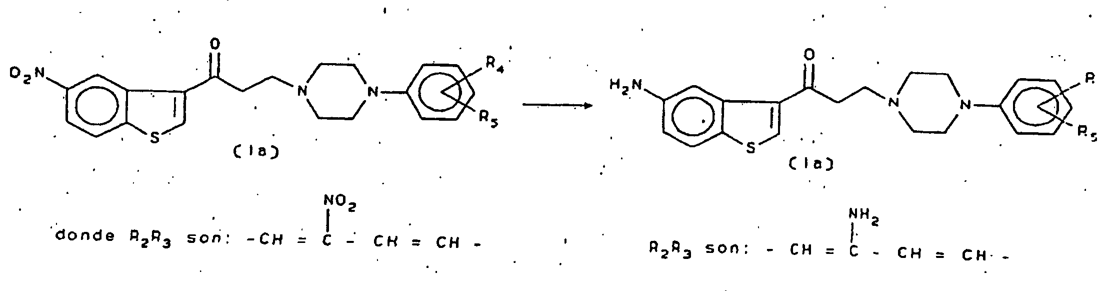

### Preparation of the formula (Ib) alcohols

The alcohol derivatives of formula (Ib) (Z is CHOH) are prepared by reducing the ketones (Ia) by the regular processes described in the literature, in accordance with the following reaction:

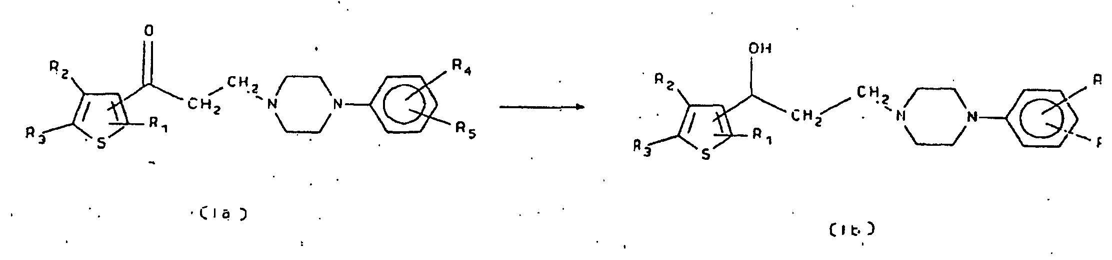

A preferred reduction process consists of using sodium hydroboride as reducing agent in an ethyl or methyl alcoholic medium and at a temperature ranging from -20°C to the reflux temperature of the corresponding alcohol. The reduction is preferably conducted at 0°C.

### Preparation of the oximes (Ic)

The oximes (Ic) (Z is C=N-OH) are prepared from the ketones (Ia) by the conventional methods described in the literature, a preferred process being the treatment of the ketones (Ia) with hydroxylamine hydrochloride in EtOH under reflux. 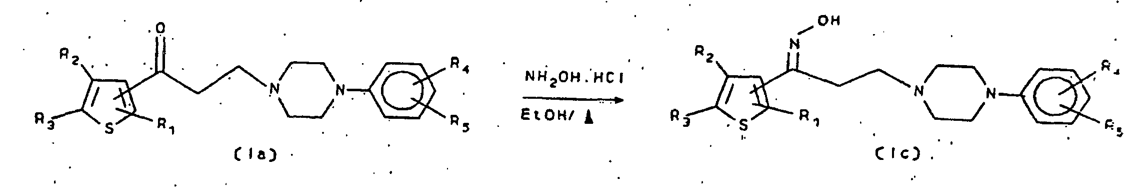 where R₁ to R₅ have the meanings given above.

### Preparation of the O-alkyl oximes (1d)

The O-alkylated oximes (Id) (Z is C=N-OR7) are prepared by reacting the ketones (Ia) with the corresponding O-alkylhydroxylamines by the general processes described in the literature, in accordance with the following reaction:

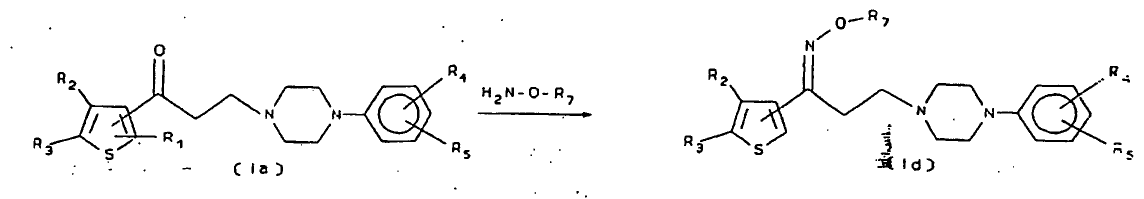

### Preparation of O-arylethers (Ie)

The arylether derivatives of formula (le) where Z is -CH(OR₆), where R₆ is an optionally substituted naphthyl or phenyl aromatic ring, are prepared by the following methods:

### Method A

Using preferably the sodium salt of the alcohols of formula (Ib) with the corresponding halogenated aryl, with the halogen displaced in the reaction being preferably an atom of fluorine.

The reaction is conducted preferably in a solvent such as dimethylformamide, dimethylacetamide or any other suitable high boiling point solvent:

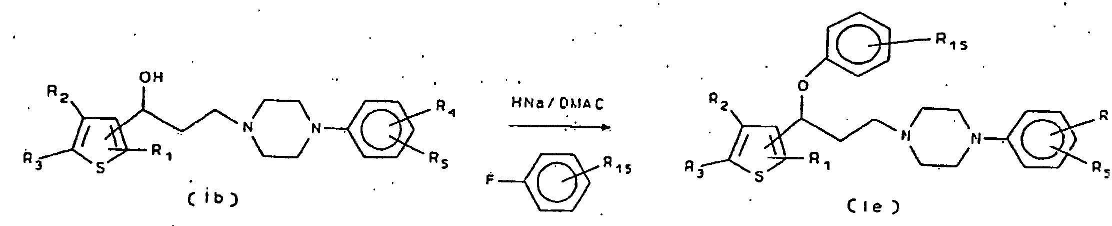

### Method B

The (Ie) derivatives may also be prepared by converting the alcohols of general formula (Ia) into derivatives with a good leaving group, such as for example a mesyl group, and reacting these intermediates with the corresponding phenolic derivative, preferably in the presence of a base such as sodium or potassium hydroxide in an alcoholic medium 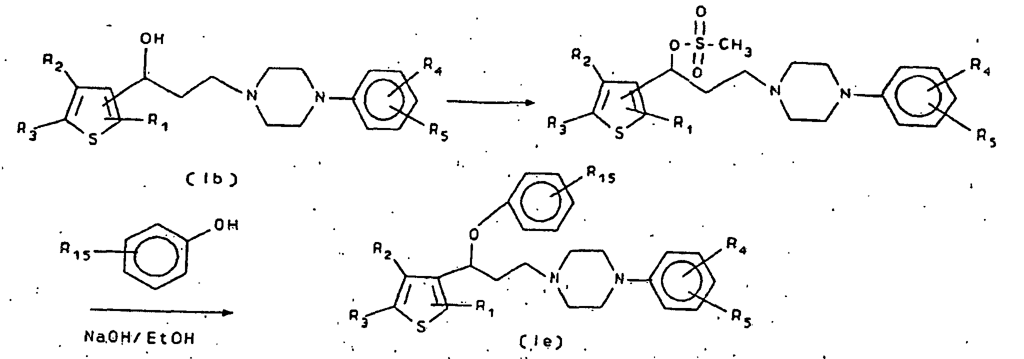 where R₁ to R₅ have the meaning given above and where R₁₅ stands for H, halogen, low molecular weight alkyl, haloalkyl, alkoxy, alkylenedioxy, nitro, cyano or a phenyl ring fused between any two positions.

### Preparation of the carbamates (If)

The carbamate derivatives of formula (If) where Z is -CH(OR₆), where R₆ is a -C(O)NR₁₃R₁₄ group, are prepared from the alcohols (Ib), by reacting them with the corresponding isocyanate. 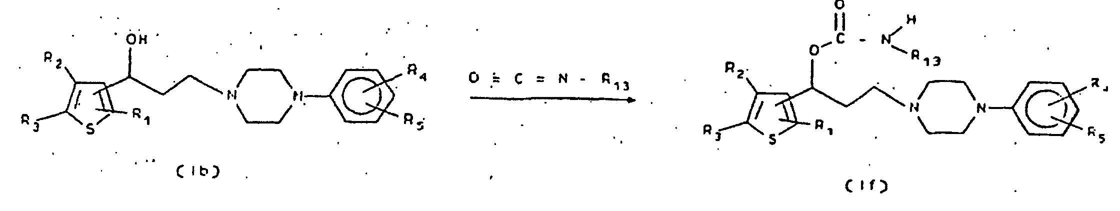 where R₁ to R₅ and R₁₃ are defined as indicated hereinbefore.

The following examples are described with an explanatory, nonlimiting purpose.

### EXPERIMENTAL

### PROCESSES OF SYNTHESIS OF FORMULA (Ia) PRODUCTS

### Process A

### Example 1: 1-(5-methylbenzo[b]thiophen-3-yl)-3-[4-(2-methoxyphenyl)piperazin-1-yl]propan-1-one dihydrochloride (VN 8312)

### 1. 3-acetyl-5-methylbenzo(b)thiophene

1 g of 5-methylbenzo(b)thiophene (6.75 x 10⁻³ moles) was stirred at 55°C with 0.8 ml of Ac₂O (8.10 x 10⁻³ moles). 0.83 ml of BF₃ Et₂O was added and stirring was continued for 8 hours. The solvent was removed in the rotary evaporator and the residue was extracted with AcOEt and H₂O. It was decanted and washed over NaHCO₃ and H₂O. It was dried over Na₂SO₄, the solvent was removed and the product was purified in successive columns of AcOEt/hexane (1:1) and toluene respectively. A product was obtained which was a mixture of the substitution isomers in positions 2- and 3- of the benzo(b)thiophene ring in a proportion of approximately 20/80 (determined by the ratios of the areas of the NMR signals), which was passed to the following reaction. Yield: 50%.

The spectroscopic characteristics of the mixture are:
IR(cm⁻¹): 1668 (mf, C=O)
¹H-NMR (CDCl₃ 200 MHz) δ (ppm): 2.47 (s, 3H, Ar-CH₃ (isomer 2)); 2.50 (s, 3H, Ar-CH₃(isomer 3)); 2.63 (s, 3H, CO-CH₃ (isomer 2 + isomer 3)); 7.22-7.27 (m, 1H, H₆) (isomer 2 + isomer 3)); 7.66 (s, 1H, H₃ (isomer 2)); 7.70-7.76 (m, 1H, H₇ (isomer 2 + isomer 3)); 7.85 (s, 1H, H₄ (isomer 2)); 8.24 (s, 1H, H₂(isomer 3)); 8.58 (s, 1H, H₄ (isomer 3)).
EM-DIP (70 eV) m/z (% Abundance): 190(M⁺)

### 2. 1-(5-methylbenzo[b]thiophen-3-yl)-3-[4-(2-methoxyphenyl)piperazin-1-yl]propan-1-one dihydrochloride

650 mg of 3-acetyl-5-methylbenzo[b]thiophene (3.42 x 10⁻³ moles) and 860 mg of 2-methoxyphenylpiperazine hydrochloride (3.76 x 10⁻³ moles) were dissolved in 15 ml of EtOH and HCl to pH = 2-3. When the mixture was under reflux, 310 mg of paraformaldehyde (10x10⁻³ moles) were added. After 24 hours stirring under reflux the reaction mixture was poured over ice and was extracted with AcOEt. It was washed with H₂O and dried over Na₂SO₄, the solvent being removed to dryness. It was purified in silica column using AcOEt/hexane (1:1) as mobile phase. The oil obtained was dissolved in ethyl ether (20 ml) and EtOH (4 ml) and precipitates as hydrochloride on adding HCl(c). Yield: 10%.
M.p. = 189-190°C.
IR(cm⁻¹): 1666 (mf, C=O); 1240 (mf, Ar-O).
¹H-NMR (DMSO-d₆ 200 MHz) δ (ppm): 2.46 (s, 3H, Ar-CH₃); 3.04-3.76 (m, 12H, -CH₂-);3.80 (s, 3H, -O-CH₃); 6.88-7.07 (m, 4H, benzene); 7.31 (dd, 1H, H₆); 7.98 (d, 1H, H₇); 8.44 (s, 1H, H₂); 9.08 (s, 1H, H₄).
EM-DIP (70 eV) m/z (% Abundance): 394 (M⁺, 81); 205 (55); 175 (100).

### Example 2: 1-(benzo[b]thiophen-3-yl)-3-[4-(4-fluor-2-methoxyphenyl)piperazin-1-yl]propan-1-one hydrochloride (VN-221F)

### 1. 2-methoxy-4-fluoroaniline hydrochloride

3.70 g of 3-methoxy-4-nitrofluorobenzene (21.6 x 10⁻³ moles) were dissolved in 40 ml of MeOH. 0.6 g of Ni-Raney and 4 ml of hydrazine hydrate were added dropwise allowing the mixture to react at 50-55°C for 2 hours. It was filtered over celite and the solvent was removed. The residue was dissolved in 200 ml of ethyl ether and 2-3 ml of HCl(c) in 40 ml of EtOH were added, thereby obtaining the product, which was collected by filtration. Yield: 65%.
M.p.: 167-168°C
IR(cm⁻¹): 3380 (m, NH₂);1245 (mf, Ar-O-).
¹H-NMR (CDCl₃ 200 MHz) δ (ppm): 3.83 (s, 3H, OCH₃); 6.98-7.24 (m, 3H, benzene); approx. 7.00 (s.a.; 2H, NH₂)
EM-DIP (70 eV) m/z (% Abundance): 141 (M⁺,6.8)

### 2. 1-(4-fluoro-2-methoxyphenyl)piperazine

9 ml of chlorobenzene and H₂O were distilled from a solution of 1.14 g of p-TosOH (6.03 x 10⁻³ moles) in 200 ml of chlorobenzene. The solution was cooled to 20°C and then 1.19 g of 4-fluor-2-methoxyaniline hydrochloride (6.7 x 10⁻³ moles) and 1.31 g of bis (2-chloroethyl)amine hydrochloride (7.36 x 10⁻³ moles) were added. The reaction was held under reflux for 72 hours after which the solvent was removed and the residue was extracted with 5 ml of NaOH 2N and 30 ml of toluene. The organic phase was washed with H₂O, was dried over Na₂SO₄ and the toluene was removed. The product was purified by silica column using dichloromethane/MeOH (9:1) as mobile phase. An oil was obtained. Yield: 25%.
IR(cm⁻¹): 3380 (m, NH₂); 1245 (mf, Ar-O-).
¹H-NMR (CDCl₃ 200 MHz) δ (ppm): 3.13 (s, 8H, CH₂); 4.00 (s, 1H, NH); 3.84 (s, 3H, OCH₃), 6.63-6.81 (m, 3H, benzene).
EM-DIP (70 eV) m/z (% Abundance): 210 (M⁺, 44); 168 (100).

### 3. 1-(benzo[b]thiophen-3-yl)-3-[4-(4-fluoro-2-methoxyphenyl)piperazin-1-yl]propan-1-one hydrochloride

A mixture of 220 mg of 3-acetylbenzo[b]thiophene (1.25 x 10⁻³ moles) and 263 mg of 1-(4-fluor-2-methoxyphenyl)piperazine (1.25 x 10⁻³ moles) in 5 ml of EtOH with sufficient HCl(c) for the pH to be equal to 2-3 was heated to reflux. Thereafter 110 mg of paraformaldehyde were added and refluxing was maintained for 8 hours. After this time, the reaction mass was allowed to cool and was poured over ice and H₂O. In this way, the hydrochloride of the product precipitated out and was collected by filtration. The filter liquors were rendered basic with NaOH 2N and extracted with AcOEt. The organic phase was washed with H₂O and with a saturated NaCl solution, was dried over Na₂SO₄ and the solvent was removed. A further fraction of the product was obtained in a silica column with AcOEt/hexane (1:1) as mobile phase. Yield: 30%.
M.p.: 199-201 °C.
IR(cm⁻¹): 1671 (f, C=O);
¹H-NMR (CDCl₃ 200 MHz) δ(ppm): 2.73 (t, 4H, (CH₂)₂-N); 2.95 (t, 2H, CO-CH₂-CH₂); 3.09 (t, 4H, (CH₂)₂-Ar); 3.25 (t, 2H, CO-CH₂)3.83 (s, 3H; O-CH₃); 6.60-6.75 (m, 3H, benzene); 7.37-7.52 (m, 2H, H₅, H₆); 7.85 (d, 1H, H₇); 8.32 (s, 1H, H₂); 8.76 (d, 1H, H₄).
EM-DIP (70 eV) m/z (% Abundance): 398 (M⁺; 40); 223 (69); 161 (100)

### Example 3. 1-(2,5-dimethylthiophen-3-yl)-3-[4-(naphthyl)piperazin-1-yl]propan-1-one hydrochloride (VN-251N) not part of the invention

### 1.- 1-(naphth-1-yl)piperazine hydrochloride

A mixture of 3 g of 1-naphthylamine (20.95 x 10⁻³ moles) with 3.68 g of bis (2-chloroethyl)amine hydrochloride (20.95 x 10⁻³ moles) and 2.90 g of Na₂CO₃ (27.36 x 10³ moles) in 47 ml of chlorobenzene was refluxed for 24 hours. The pH of the medium was controlled to keep it basic; to this end, Na₂CO₃ was added when necessary. At the end of this time, the reaction mass was diluted with H₂O and the two phases were separated. The aqueous phase was extracted twice with 100 ml of AcOEt. The combined organic phases were washed with H₂O, were dried over Na₂SO₄ and the solvent was removed. The product was purified in a silica column, using dichloromethane/MeOH (9:1) as mobile phase. The product was dissolved in acetone and HCl(c) was added to precipitate the hydrochloride. Yield: 35%.
M.p.: 285°C.
IR(cm⁻¹): 3375 (m, NH).
¹H-NMR (DMSO-d₆ 200 MHz) δ (ppm): 3.25 (s, 4H,(CH₂)₂-NH); 3.39 (s, 4H,(CH₂)₂-N-Ar); 7.19 (dd, 1H, H₆); 7.44-7.58 (m, 3H, H₅, H₇, H₈); 7.68 (d, 1H, H₄); 7.94 (dd, 1H, H₃); 8.18 (dd, 1H, H₂; 9.63 (s.a., 1H, NH).
EM-DIP(70 eV)m/z (%Abundance): 210 (M⁺; 54.3),170 (100)

### 2. 1-(2,5-dimethylthiophen-3-yl)-3-[4-(naphthyl)piperazin-1-yl]propan-1-one hydrochloride

A mixture of 1.1 g of 3-acetyl-2,5-dimethylthiophene (7.13 x 10⁻³ moles) and 1.50 g of 1-naphthylpiperazine hydrochloride (6.04 x 10⁻³ moles) was heated under reflux in 40 ml of EtOH with sufficient HCl(c) for the pH to be 1-2. Thereafter 0.45 g of paraformaldehyde was added and the reflux was maintained until no further evolution of the reaction was appreciated by TLC (thin layer chromatography). After this time, the reaction mixture was allowed to cool and was poured over ice and H₂O. The aqueous phase was extracted with AcOEt, was washed with H₂O, was dried over Na₂SO₄ and the solvent was removed to dryness. The product was purified by formation of the hydrochloride and recrystallisation of the latter in isopropanol. Yield: 29%.
M.p.:250-252°C.
IR(cm⁻¹): 1672 (f, C=O)
¹H-NMR (DMSO-d₆ 200 MHz) δ (ppm): 2.34 (s, 3H, CH₃-C₅); 2.62 (s, 3H, CH₃-C₂); 2.23-2.38 (m, 6H, (CH₂)₃-N); 2.54-2.63 (m, 6H, CO-CH₃, (CH₂)₂-N-Ar); 7.08-7.23 (m, H₂ , H₃); 7.29 (s, 1H; thiophene); 7.32-7.46 (m, 2H, H₆ , H₇); 7.57 (d, 1H, H₄); 7.79 (dd, 1H, H₅); 7.98 (dd, 1H, H₈).
EM-DIP (70 eV) m/z (% Abundance): 378 (M⁺; 59); 225 (100); 139 (98).

Following the process described in Examples 1 to 3, the compounds listed below were prepared:

### Example 4: 1-(benzo[b]thiophen-3-yl)-3-[4-(-2 methoxyphenyl)piperazin-1-yl]propan-1-one hydrochloride. (VN-2212)

M.p.: 214-215°C.
IR(cm⁻¹): 1670 (f, C=O); 1243 (mf, Ar-O)
¹H-NMR (CDCl₃ 200 MHz) δ (ppm): 2.04-2.16 (m, 2H, CO-CH₂); 2.65-2.83 (m, 6H, (CH₂)₃-N); 2.97 (t, 4H, (CH₂)₂-N-Ar); 5.36 (dd, 1H, CHOH); 6.70 (s.a., 1H, OH); 6.95-7.00 (s.a., 4H, benzene); 7.37-7.59 (m, 2H, H₅,H6); 8.11 (d, 1H, H₄); 8.61 (d, 1H, H₇); 9.14 (s, 1H, H₂).
EM-DIP (70 eV) m/z (% Abundance): 398(M⁺; 40); 223(69); 161 (100)

### Example 5: 1-(benzo[b]thiophen-3-yl)-3-[4-(2-hydroxyphenyl)piperazin-1-yl]propan-1-one hydrochloride. (VN-221H)

M.p.: 292-294°C
IR(cm⁻¹): 3235 (m, OH); 1659 (f, C=O); 1255 (m, Ar-O)
¹H-NMR (DMSO-d₆ 200 MHz) 8 (ppm): 3.06 (t, 2H, CO-CH₂-CH₂); 3.26-3.76 (m, 8H, (CH₂)₄-N); 3.77 (t, 2H, CH₂-CO); 6.74-6.93 (m, 4H, benzene); 7.45-7.59 (m, 2H, H₅,H₆); 8.13 (dd, 1H, H₇); 8.61 (dd, 1H, H₄); 9.12 (s, 1H, H₂); 9.38 (s, 1H, OH).
EM-DIP (70 eV) m/z (% Abundance): 398(M⁺; 40); 223(69); 161(100)

### Example 6: 3-[4-(2-chlorophenyl)piperazin-1-yl]-1-(thiophen-3-yl)propan-1-one hydrochloride. (VN-2110) not part of the invention

M.p.: 174-176°C
IR(cm⁻¹): 1676 (f, C=O).
¹H-NMR (DMSO-d₆ 200 MHz) δ (ppm): 3.20-3.63 (m, 12H, CH₂); 7.07-7.48 (m, 4H, benzene); 7.55 (dd; 1H, H₅); 7.69 (d, 1H, H₄); 8.61 (d, 1H, H₂).
EM-DIP(70 eV)m/z (% Abundance): 334(M⁺; 12); 209(60); 111 (100).

### Example 7: 3-[4-(2-methoxyphenyl)piperazin-1-yl]-1-thiophen-3-yl)propan-1-one hydrochloride. (VN-2112) not part of the invention

M.p.: 145-149°C
IR(CM¹): 1672 (f, C=O); 1242 (mf, Ar-O).
¹H-NMR (DMSO-d₆ 200 MHz) δ (ppm): 3.17-3.64 (m, 12H, CH₂); 6.93-7.02 (m, 4H, benzene); 7.55 (dd, 1H, H₅); 7.69 (d, 1H, H₄); 8.61 (d, 1H, H₂).
EM-DIP(70 eV)m/z (% Abundance):330(M⁺; 70); 205(81); 111(100).

### Example 8: 3-[4-(3-trifluorophenyl)piperazin-1-yl]-1-(thiophen-3-yl)-propan-1-one oxalate. (VN-2113) not part of the invention

M.p.: 110°C.
IR (cm⁻¹): 1645 (f, C=O).
¹H-NMR (DMSO-d₆ 200 MHz) δ (ppm): 2.85 (s, 4H, (CH₂)₂N-Ar); 3.30 (s, 6H, (CH₂)₃N); 3.64 (s, 2H, CH₂-CO); 7.06-7.41 (m, 4H, benzene); 7.48 (dd, 1H, H₅); 7.67 (d, 1H, H₄); 8.35 (d, 1H, H₂)
EM-DIP (70 eV) m/z (% Abundance): 380(M⁺; 29); 229(46); 111(100)

### Example 9: 3-[4-(4-chlorophenyl)piperazin-1-yl]-1-(thiophen-3-yl)propan-1-one hydrochloride. (VN-2115) Not part of the invention

M.p.: 148-149°C.
IR (cm⁻¹): 1679 (f. C=O).
¹H-NMR (DMSO-d₆ 200 MHz) δ (ppm): 3.12-3.17 (m, 4H, (CH₂)₂-N), 3.46-3.49 (m, 2H, CO-CH₂-CH₂-N); 3.60(s, 4H, (CH₂)₂-N-Ar); 3.82-3.87 (m, 2H, CH₂-CO); 7.03 (d, 2H, H_{2'},H_{6'}); 7.29 (d, 2H H_{3'}H_{5'}); 7.55 (dd, 1H, H₄); 7.68 (dd, 1H, H₅); 8.60 (dd, 1H, H₂).
EM-DIP (70 eV) m/z (% Abundance): 334(M⁺; 6,1); 209(74); 111(100)

### Examale 10: 1-(2,5-dimethylthiophen-3-yl)-3-[4-(2-hydroxyphenyl)piperazin-1-yl]propan-1-one hydrochloride. (VN-251H) not part of the invention

M.p.: 202-203°C.
IR (cm⁻¹): 3234 (m, OH); 1665 (f, C=O).
¹H-NMR (DMSO-d₆ 200 MHz) δ (ppm): 2.40 (s, 3H, CH₃-C₅); 2.62 (s, 3H, CH₃-C₂); 3.00-3.60 (m, 12H, CH₂); 6.71-6.90 (m, 4H, benzene); 7.28 (s, 1H, H₄); 9.37 (s, 1H, OH)
EM-DIP (70 eV) m/z (% Abundance): 344(M⁺; 41); 191(57); 125(100).

### Example 11: 1-(2,5-dimethylthiophen-3-yl)-3-[4-(2-methoxyphenyl)piperazin-1-yl]propan-1-one hydrochloride. (VN-2512) not part of the invention

M.p.: 185°C.
IR (cm⁻¹): 1665 (f., C=O); 1246 (mf., Ar-O).
¹H-NMR (DMSO-d₆ 200 MHz) δ (ppm): 2.40 (s, 3H, CH₃-C₅); 2.62 (s, 3H, CH₃-C₂); 3.08-3.55 (m, 12H, CH₂); 3.80 (s, 3H, OCH₃); 6.93-6.99 (m, 4H, benzene); 7.28 (s, 1H, H₄)
EM-DIP (70 eV) m/z (% Abundance): 358(M⁺ 100); 205(96).

### Example 12: 1-(2,5-dimethylthiophen-3-yl)-3-[4-(4-fluor-2-methoxyphenyl)piperazin-1-yl]propan-1-one dihydrochloride. (VN-251F) not part of the invention

M.p.: 175°C
IR (cm⁻¹): 1664 (f, C=O); 1229 (f, Ar-O).
¹H-NMR (DMSO-d₆ 200 MHz) δ (ppm): 2,40 (s, 3H, CH₃-C₅); 2,62 (s, 3H, CH₃-C₂); 3,00-3,53 (m, 12H, CH₂); 3,79 (s, 3H, OCH₃);6,78-7,00 (m, 3H, benzene); 7,28 (s, 1H, H₄).
EM-DIP (70 eV) m/z (% Abundance): 376(M⁺; 44); 223(88); 139(100),

### Example 13: 1-(5-methylthiophen-2-yl)-3-[4-(2-methoxyphenyl)piperazin-1-yl]propan-1-one hydrochloride. (VN-2312) not part of the invention

M.p.: 167-168°C.
IR (cm⁻¹): 1655 (f. C=O); 1243 (mf., Ar-O).
¹H-NMR (DMSO-d₆ 200 MHz) δ (ppm): 2.53 (s, 3H, CH₃); 3.19-3.62 (m, 12H, CH₂); 3.80 (s, 3H, OCH₃); 6.93-6.99 (m, 5H, benzene, H₄); 7.91 (d, 1H, H₃)
EM-DIP (70 eV) m/z (% Abundance): 344(M⁺ 52); 205(53); 125(100).

### Example 14: 3-[4-(2-methoxyphenyl)piperazin-1-yl]-1-(5-nitrothiophen-1-yl)propan-1-one hydrochloride. (VN-2412) not part of the invention

M.p.: 178°C
IR (cm⁻¹): 1655 (f. C=O); 1243 (mf, Ar-O).
¹H-NMR (DMSO-d₆ 200 MHz) δ (ppm): 3.01-3.35 (m, 6H (CH₂)₃-N); 3.48-3.64 (m, 4H, (CH₂)₂-N-Ar); 3.73-3.79 (t, 2H, CH₂-CO); 3.79 (s, 3H, OCH₃); 6.93-6.99 (m, 4H, benzene); 8.11 (d, 1H, H₃); 8.26 (d, 1H, H₄):
EM-DIP (70 eV) m/z (% Abundance): 375(M⁺ 0,5); 150(100)

### Example 15: 3-[4-(1-naphthyl)piperazin-1-yl]-1-(3-thiophenyl)propan-1-one hydrochloride. (VN-211N) not part of the invention

M.p.: 258-260°C
IR (cm⁻¹): 1673 (f, C=O);
¹H-NMR (DMSO-d₆ 200 MHz) δ (ppm): 3.29-3.70 (m, 12H, CH₂); 7.17 (d, 1H H_{2'}); 7.42-7.58 (m, 5H, H_{3'}+H_{4'}+H_{5'}+H_{6'}+H_{7'}); 7.67 (d, 1H, H_{8'}); 7.92 (dd, 1H, H₅); 8.13 (d, 1H, H₄); 8.64 (d, 1H, H₂)
EM-DIP (70 eV) m/z (% Abundance): 350(M⁺; 47); 212(47); 111(100)

### Example 16: 1-(3,5-dimethylbenzo[b]thiophen-2-yl)-3-[4-(2-methoxyphenyl)piperazin-1-yl]propan-1-one hydrochloride. (VN-7112)

M.p.: 86-87°C.
IR (KBr)(cm⁻¹): 1671 (f, C=O).
¹H-NMR (DMSO-d₆, 200 MHz) δ: 2.42 (s, 3H, CH₃, C₅); 2.67 (s, 3H, CH₃,C₃); 2.97-3.21 (m, 8H, (CH₂)₃-N+CH₂CO); 3.34-3.62 (m, 4H, (CH₂)₂-N-Ar); 6.81-7.02 (m, 4H, benzene); 7.36 (d, 1H, H₆); 7.75 (s, 1H, H₄); 7.85 (d, 1H, H₇).
EM-DIP (70 eV) m/z (% Abundance): 408(M⁺,1); 190(7); 189(42); 216(79).

### Example 17: 1-(3-methylbenzo[b]thiophen-2-yl)-3-[4-(2-methoxyphenyl)piperazin-1-yl]propan-1-one hydrochloride. (VN-7012)

M.p.: 190-193°C.
IR (KBr) (cm⁻¹): 1723 (f, C=O).
¹H-NMR (DMSO-d₆, 200 MHz) δ: 2.63 (s, 3H, CH₃,C₃); 2.77-3.11 (m, 8H, (CH₂)₃-N+CH₂CO); 3.35-3.52 (s.a., 4H, (CH₂)₂-N-Ar); 3.79 (s, 3H, CH₃O); 6.78-7.10 (m, 4H, benzene); 7.50-7.58 (m, 2H, H₆+H₅); 8.05 (d, 2H, H₄+H₇)
EM-DI P (70 eV) m/z (% Abundance): 394(M⁺,1); 175(28); 150(100).

### Example 18: 1-(3-methylbenzo[b]thiophen-2-yl)-3-[4-(2-hydroxyphenyl)piperazin-1-yl]propan-1-one hydrochloride. (VN-701H)

M.p.: 207-210°C.
IR (KBr) (cm⁻¹): 3406 (m, C-OH); 1775 (f, C=O).
¹H-NMR (DMSO-d₆, 200 MHz) δ: 2.77 (s, 3H, CH₃, C₃); 3.06-3.77 (m, 12H, CH₂); 6.71-7.91 (m, 4H, benzene); 7.47-7.62 (m, 2H, H₆+H₅); 8.03 (d, 2H, H₄+H₇); 9.36 (s, 1H, OH)
EM-DIP (70 eV) m/z (% Abundance): 380(M⁺, 5); 175(56); 147(63); 120(100).

### Example 19: 1-(3-methylthiophen-2-yl)-3-[4-(2-methoxyphenyl)piperazin-1-yl]propan-1-one hydrochloride. (VN-2712) not part of the invention

M.p.:138°C.
IR (KBr) (cm⁻¹): 1664 (f, C=O).
¹H-RMN (DMSO-d₆, 200 MHz) δ: 2.51 (s, 3H, CH₃); 2.95-3.56 (m, 12H, CH₂); 3.79 (s, 3H, CH₃O); 6.90-7.01 (m, 4H, benzene); 7.2 (d, 1H, H₄); 7.88 (d, 1H, H₅)
EM-DIP (70 eV) m/z (% Abundance): 344(M⁺,6); 205(55); 125(100).

### Process B

### Example 20: 3-[4-(2-methoxyphenyl)piperazin-1-yl]-1-(5-nitrobenzo[b]thiophen-3-yl)propan-1-one hydrochloride. (VN-8012)

### 1. 5-nitrobenzo[b]thiophen-2-carboxylic acid ethyl ester

15 g (80.8 x 10⁻³ moles) of 2-chloro-5-nitrobenzaldehyde dissolved in EtOH were added dropwise to a solution of 19.41 g (80.8 x 10⁻³ moles) of Na₂S·9H₂O in EtOH at 40°C. It was held under reflux for 2 hours and thereafter 9 ml (80.8 x 10⁻³ moles) of ethyl bromoacetate were added. After 2 hours at 50°C, Et₃N was added to pH = 8-9. The mixture was left to react overnight at room temperature. The yellow product was collected by filtration. A further amount of product precipitated out on adding H₂O to the filtrate. It was purified by recrystallisation in hexane/AcOEt. Yield: 75%.
M.p.: 165°C
IR (cm⁻¹): 1714 (mf, C=O); 1533-1505, 1334-1258 (mf, NO₂)
¹H-NMR (CDCl₃, 200 MHz) δ (ppm): 1.36 (t, 3H, CH₃-CH₂-O); 4.38 (c, 2H, CH₃-CH₂-O); 8.28 (s, 2H, H₆+H₇); 8.36 (s, 1H, H₃); 8.92 (s, 1H, H₄)
EM-DIP (70 eV) m/z (% Abundance): 251 (82); 206(100); 160(45)

### 2. 5-nitro-benzo[b]thiophen-2-carboxylic acid

10 g (39.8 x 10⁻³ moles) 5-nitrobenzo[b]thiophen-2-carboxylic acid ethyl ester in 250 ml of EtOH and 60 ml of H₂O were reacted with 3.8 g (67.8 x 10⁻³ moles) of KOH at 60°C for 2 hours, after which the potassium salt of the product was collected by filtration. A further fraction of the product was collected on adding isopropanol to the filtrate. The salt was dissolved in water and after acidulating the solution with HCl(c), the protonated form of the acid was precipitated out. It was purified by recrystallisation in H₂O/EtOH. Yield: 85%.
M.p.: 238°C
IR (cm⁻¹): 1688 (mf, C=O); 1532, 1357-1307 (mf, NO₂)
¹H-NMR (DMSO-d₆; 200 MHz) δ (ppm): 8.30 (s, 2H, H₆+H₇); 8.32 (s, 1H, H₃); 8.96 (s, 1H, H₄)
EM-DIP (70 eV) m/z (% Abundance): 223 (100)

### 3. 5-nitrobenzo[b]thiophene

5 g (22.4 x 10⁻³ moles) of 5-nitro-benzo[b]thiophen-2-carboxylic acid in 105 ml of quinoline were heated, together with 5.2 g of powdered copper to a temperature of 180-190°C for 45 minutes. The reaction mixture was filtered under vacuum and the filter was washed with ethyl ether. The mixture was extracted with ethyl ether twice, the phases being allowed to decant well, and the ethereal phase was washed with HCl 6N until no quinoline remains were left. It was dried over Na₂SO₄, the solvent was removed and the product was purified by recrystallisation in hexane/isopropanol. Yield: 65%.
M.p.: 150°C.
IR (cm⁻¹): 1714 (mf, C=O); 1533-1505, 1334-1258 (mf, NO₂)
¹H-NMR (CDCl₃, 200 MHz) δ (ppm): 7.43 (d, 1H, H₃); 7.59 (d, 1H, H₂); 7.91 (d, 1H, H₇); 8.13 (dd, 1H, H₆); 8.65 (d, 1H, H₄)
EM-DIP (70 eV) m/z (% Abundance): 179(100); 133(68).

### 4. 3-chloro-1-(5-nitro-benzo[b]thiophen-3-yl)-propan-1-one

A solution of 1 g (5.58 x 10⁻³ moles) of 5-nitrobenzo[b]thiophene and 0.65 ml (6.64 x 10⁻³ moles) of 2-chloropropionyl chloride dissolved in 40 ml of dry chloroform was added dropwise over 650 mg of aluminium trichloride, dissolved in 20 ml dry chloroform under a nitrogen atmosphere. The mixture was left to react for 24 hours at room temperature and an additional amount of 650 mg of aluminium trichloride and 0.65 ml of 2-chloropropionyl chloride was added. After 48 hours reaction 100 ml of HCl 1.5N were added, followed by decantation and the organic phase was subsequently washed with a dilute solution of NaHCO₃, with H₂O and with a saturated solution of NaCl. It was dried over Na₂SO₄, the solvent was removed and it was purified in a silica column using hexane/toluene (25:75) as mobile phase. Yield: 30%.
M.p.: 128°C.
IR (cm⁻¹): 1670 (mf, C=O); 1510, 1335 (mf, NO₂)
¹H-NMR (CDCl₃, 200 MHz) δ (ppm): 3.51 (t, 2H, CH₂-C=O); 3.98 (t, 2H, CH₂-Cl); 7.99 (d, 1H, H₇); 8.29 (dd, 1H, H₆); 8.49 (s, 1H, H₂); 9.64 (d, 1H, H₄)
EM-DIP (70 eV) m/z (% Abundance): 269(17); 206(100)

### 5. 3-[4-(2-methoxyphenyl)piperazin-1-yl]-1-(5-nitrobenzo[b]thiophen-3-yl)propan-1-one hydrochloride.

To 600 mg of (5-nitrobenzo[b]thiophen-3-yl)-3-chloropropan-1-one (2.26 x 10⁻³ moles) dissolved in 30 ml of THF were added 1.3 g of 2-methoxyphenylpiperazine (6.78 x 10⁻³ moles) and 244 mg of Na₂CO₃ (2.26 x 10⁻³ moles). After stirring for 72 hours at room temperature completion of the reaction was checked by TLC. The THE was removed and the residue was poured over water/ice, was extracted with AcOEt, the organic phase was washed with H₂O and a saturated solution NaCl and the solvent was removed. The residue was purified in a silica column using AcOEt/hexane (1:1) as mobile phase. Yield: 75%.
M.p.: 208-210°C
IR (cm⁻¹): 1679 (mf, C=O); 1516-1333 (mf, NO₂); 1250 (mf, Ar-O-).
¹H-NMR (DMSO-d₆ 200 MHz) 8 (ppm): 3.01-3.76 (m, 12H, -CH₂-); 3.80 (s, 3H, -O-CH₃); 6.86-7.03 (m, 4H, benzene); 8.30 (dd, 1H, H₆); 8.43 (d, 1H, H₇); 9.32 (s, 1H, H₂); 9.40 (d, 1H, H₄).
EM-DIP (70 eV) m/z (% Abundance): 425(M⁺; 10); 206(68); 150(100).

### Process C

### Example 21 1-(5-aminobenzo[b]thiophen-3-yl)-3-[4-(2-methoxyphenyl)piperazin-1-yl]propan-1-one dihydrochloride (VN-8112)

200 mg of 1-(5-nitrobenzo[b]thiophen-3-yl)piperazin-1-yl]propan-1-one dihydrochloride, obtained according to the process described in Example 8, were dissolved in 20 ml of THF with 100 mg of Ni-Raney and were subjected to a pressure of 50 p.s.i. H₂ and to a temperature of 40°C for 2 hours, after which a further 100 mg of Ni-Raney were added: After 24 hours during which the reaction was kept under regular stirring, the disappearance of the starting product was observed in TLC using dichloromethane/MeOH (9:1). It was filtered over celite, the solvent was quickly removed to avoid possible oxidation and the residue was dissolved in 10 ml of ethyl ether and 2 ml of EtOH. The hydrochloride of the product precipitated on adding 0.1 ml of HCl(c). The solid was washed with hot acetone and the product was obtained by filtration. Yield: 57%.
M.p.: 200-201°C
IR (cm⁻¹): 3354 (m, NH₂); 1667 (mf, C=O); 1245 (mf, Ar-O-).
¹H-NMR (DMSO-d₆200 MHz) δ (ppm): 3.04-3.75 (m, 12H, -CH₂-); 3.79 (s, 3H, -O-CH₃); 6.89-7.06 (m, 4H, benzene); 7.48 (dd, 1H, H₆); 8.21 (d, 1H, H₇); 8.60 (d, 1H; H₄); 9.23 (s, 1H, H₂).
EM-DIP (70 eV) m/z (% Abundance): 395 (M⁺; 2.2); 176 (40); 150 (100).

### PROCESS OF SYNTHESIS OF FORMULA (Ib) PRODUCTS

### Example 22: 1-(benzo[b]thiophen-3-yl)-3-[4-(2-methoxyphenyl)piperazin-1-yl]propan-1-ol (VN-2222)

To 500 mg of 1-(benzo[b]thiophen-3-yl)-3-[4-(2-methoxyphenyl)piperazin-1-yl]propan-1-one hydrochloride (1.20 x 10⁻³ moles) in 30 ml of MeOH a 0°C was added NaBH₄ for about 20 min until the reaction ceased to evolve. After two hours 50 ml of H₂O were added to the medium, it was stirred for a few minutes and was extracted twice with 200 ml of AcOEt. The organic phase was washed three times with H₂O, was dried over Na₂SO₄ and the solvent was removed. It was purified in silica column with AcOEt/hexane (1:1). The free base was thus obtained in form of a white solid. Yield: 30%. Two polymorphic forms of this product were obtained, the melting points of which were 108°C and 120°C respectively.
IR (cm⁻¹): 3220 (m, OH); 1243 (mf, Ar-O-)
¹H-NMR (CDCl₃ 200 MHz) δ (ppm): 2.06-2.13 (m, 2H, CH₂-CHOH); 2.68-2.88 (m, 6H, (CH₂)₃-N); 3.16 (s.a., 4H, (CH₂)₂-N-Ar); 3.87 (s, 3H, OCH₃); 6.89-7.00 (m, 4H, benzene) 7.21-7.39 (m, 2H, H₅ + H₆); 7.42 (s, 1H, H₂); 7.77-7.86 (m, 2H, H₄ + H₇).
EM-DIP (70 eV) m/z (% Abundance): 368(M⁺; 6.8); 120(100)

Using the process described in the Example 22 and starting out from the corresponding ketches described in Examples 1 to 21 the following products were prepared:

### Example 23: 3-[4-(2-methoxyphenyl)piperazin-1-yl]-1-(5-nitrobenzo[b]thiophen-3-yl)propan-1-ol monohydrate dihydrochloride (VN-8022)

M.p.: 130-131°C
IR (cm⁻¹): 3404 (m, OH); 1510-1330 (mf, NO₂); 1245 (mf, Ar-O-).
¹H-NMR (DMSO-d₆ 200 MHz) δ (ppm): 2.09 (m, 2H, CHOH-CH₂); 3.06-3.29 (m, 6H, (CH₂)₃-N); 3.46-3.61 (m, 4H, (CH₂)₂N-Ar); 3.79 (s, 3H, OCH₃); 5.17 (dd, 1H, CHOH); 6.93-6.99 (m, 4H, benzene); 7.96 (s, 1H, H₂); 8.20 (dd, 1H, H₆); 8.31 (d, 1H, H₇); 8.90 (d, 1H, H₄).
EM-DIP (70 eV) m/z (% Abundance): 427 (M⁺; 61); 219 (100).

### Example 24: 1-(5-methylbenzo[b]thiophen-3-yl)-3-[4-(2-methoxyphenyl)piperazin-1-yl]propan-1-ol dihydrochloride (VN-8322)

M.p.: 109-111°C.
IR (cm⁻¹): 3425 (m, OH); 1245 (mf, Ar-O-).
¹H-NMR (CDCl₃ 200 MHz) δ (ppm): 2.08 (dd, 2H, CO-CH₂); 2.43 (s, 3H, CH₃); 2.65-2.88 (m, 6H, (CH₂)₃-N); 3.14 (s.a. 4H, (CH₂)₂-N-Ar); 3.85 (s, 3H, O-CH₃); 5.29 (t, 1H, CHOH); 6.70 (s.a. 1H, OH); 6.83-7.08 (m, 4H, benzene); 7.15 (dd, 1H, H₆); 8.21 (d, 1H, H₇); 8.60 (d, 1H, H₄); 9.23 (s, 1H, H₂).
EM-DIP (70 eV) m/z (% Abundance): 396(M⁺; 27); 205(100).

### Example 25: 1-(benzo[b]thiophen-3-yl)-3-[4-(4-fluoro-2-methoxyphenyl)piperazin-1-yl]propan-1-ol hydrochloride. (VN-222F)

M.p.: 109-111°C.
IR (cm⁻¹): 3425 (m, OH); 1245 (mf, Ar-O-)
¹H-NMR (CDCl₃ 200 MHz) δ (ppm): 2.08 (dd, 1H, CO-CH₂); 2.43 (s, 3H, CH₃); 2.65-2.88 (m, 6H, (CH₂)₃-N); 3.14 (s.a. 4H, (CH₂)₂-N-Ar); 3.85 (s, 3H, O-CH₃); 5.29 (t, 1H, CHOH); 6.70 (s.a. 1H, OH); 6.83-7.08 (m, 4H, benzene); 7.15 (dd, 1H, H₆); 8.21 (d, 1H, H₇); 8.60 (d, 1H, H₄); 9:23 (s, 1H, H₂)
EM-DIP (70 eV) m/z (% Abundance): 396(M⁺; 27); 205(100)

### Example 26: 1-(benzo[b]thiophen-3-yl)-3-[4-(2-hydroxyphenyl)piperazin-1-yl]propan-1-ol (VN-222H)

M.p.: 109-111°C.
IR (cm⁻¹): 3220 (m, OH); 1243 (mf, Ar-O-)
¹H-NMR (CDCl₃ 200 MHz) δ (ppm): 2.04-2.16 (m, 2H, CHOH-CH₂); 2.65-2.83 (m, 6H, (CH₂)₃-N); 2.97 (t, 4H,(CH₂)₂-N-Ar); 5.36 (dd, 1H, CHOH); 6.70 (s.a. 1H, OH); 6.83-7.19 (m, 4H, benzene); 7.30-7.41 (m, 2H, H₅ + H₆); 7.44 (s, 1H, H₂); 7.78-7.89 (m, 2H, H₄ + H₇)
EM-DIP (70 eV) m/z (% Abundance): 368(M⁺; 6.8); 120(100)

### Example 27: 1-(benzo[b]thiophen-3-yl)-3-[4-(4-chlorophenyl)piperazin-1-yl]propan-1-ol. (VN-2225)

M.p.: 148-150°C.
IR (cm⁻¹): 3150 (m, OH); 1230 (mf, Ar-O-)
¹H-NMR (CDCl₃ 200 MHz) δ (ppm): 1.99 (t, 2H, CHOH-CH₂); 2.52-2.70 (m, 6H, (CH₂)₃-N); 3.10 (t, 4H, (CH₂)₂-N-Ar); 5.23 (t, 1H, CHOH); 6.72 (d, 2H, H_{2'},+ H₆'); 7.11 (d, 2H, H_{3'}+H_{4'}); 7.25 (d, 2H, H₅+H₆); 7.32 (s, 1H, H₂); 7.67-7.78 (m, 2H, H₄+H₇)
EM-DIP (70 eV) m/z (% Abundance): 386(M⁺, 38); 209(100).

### Example 28: 3-[4-(2-chlorophenyl)piperazin-1-yl]-1-(thiophen-3-yl)propan-1-ol. (VN-2120) not part of the invention

M.p.: 125-126°C
IR (cm⁻¹): 3243 (m, OH).
¹H-NMR (CDCl₃ 200 MHz) δ (ppm): 1.93-2.03 (m, 2H, CH₂-CHOH); 2.67-2.93 (m, 6H, (CH₂)₃-N); 3.10 (t, 4H, (CH₂)₂-N-Ar); 5.03 (dd, 1H, CHOH); 6.93-7.37 (m, 7H, Ar-H).
EM-DIP (70 eV) m/z (% Abundance): 336 (M⁺; 13); 209 (100).

### Example 29: 3-[4-(2-methoxyphenyl)piperazin-1-yl]-1-(thiophen-3-yl)propan-1-ol dihydrochloride. (VN-2122). not part of the invention

M.p.: 152-153°C
IR (cm⁻¹): 3414 (f, OH); 1243 (f, Ar-O).
¹H-NMR (CDCl₃ 200 MHz) δ (ppm): 2.07- 2.14 (m, 2H, CH₂-CHOH); 3.14 (s, 6H, (CH₂)₃-N); 3.48 (t, 4H, (CH₂)₂-N-Ar); 3.78 (t, 3H, OCH₃); 4.72 (dd, 1H, CHOH); 6.95 (d, 4H, benzene); 7.13 (d, 1h, H₄); 7.36 (d, 1H, H₂); 7.49 (dd, 1H, H₅).
EM-DIP (70 eV) m/z (% Abundance): 332(M⁺; 35); 205(100).

### Example 30: 3-[4-(4-chlorophenyl)piperazin-1-yl]-1-(thiophen-3-yl)propan-1-ol. (VN-2125) not part of the invention

M.p.: 140 - 142°C
IR (cm⁻¹): 3277 (m, OH); 809 (m, p-substitution).
¹H-NMR (CDCl₃ 200 MHz) δ (ppm): 1.93-1.99 (m, 2H, CH₂-CHOH); 2.57-2.80 (m, 6H, (CH₂)₃-N); 3.18 (t, 4H, (CH₂)₂-N-Ar); 5.02 (dd, 1H, CHOH); 6.82 (d, 2H, H₃, H₅); 7.03 (d, 1H, H₄); 7.20 (d, 2H, H_{2'}, H_{6'}); 7.26-7.30 (m, 2H, H₂, H₅).
EM-DIP (70 eV) m/z (% Abundance): 336 (M⁺;14); 209 (100)

### Example 31: 3-[4-(2-methoxyphenyl)piperazin-1-yl]-1-(thiophen-2-yl)propan-1-ol dihydrochloride. (VN-2022) not part of the invention

M.p.: 127-128°C.
IR (cm⁻¹): 3342 (f, OH); 1263 (f, Ar-O).
¹H-NMR (CDCl₃ 200 MHz) δ (ppm): 2.22 (s, 2H, CH₂-CHOH); 3.18 (s, 6H, (CH₂)₃-N); 3.50 (t, 4H, (CH₂)₂-N-Ar); 3.79 (s, 3H, OCH₃); 4.93 (dd, 1H, CHOH); 6.90-7.01 (m, 6H, benzene + H₃+ H₄); 7.42 (d, 1H, H₅).
EM-DIP (70 eV) m/z (% Abundance): 332(M⁺; 53); 205(100).

### Example 32: 1-(2,5-dimethylthiophen-3-yl)-3-[4-(2-methoxyphenyl)piperazin-1-yl]propan-1-ol hydrochloride. (VN-2522) not part of the invention

M.p.: 158°C.
IR (cm⁻¹): 3371 (f, OH); 1260 (f, Ar-O).
¹H-NMR (CCDl₃ 200 MHz) δ (ppm): 1.90-2.07 (m, 2H, CH₂-CHOH); 2.35 (s, 3H, CH₃-C₅); 2.39 (s, 3H, CH₃-C₂); 2.58-3.00 (m, 6H, (CH₂)₃-N); 3.13 (s.a., 4H, (CH₂)₂-N-Ar); 3.86 (s, 3H, OCH₃); 4.92 (dd, 1H, CHOH); 6.71 (s, 1H, H₄); 6.84-7.01 (m, 4H, benzene).
EM-DIP (70 eV) m/z (% Abundance): 360(M⁺; 61.7); 205(100)

### Example 33: 1-(2,5-dimethylthiophen-3-yl)-3-[4-(2-hyrdroxyphenyl)piperazin-1-yl]-propan-1-ol. (VN-252H) not part of the invention

M.p.: 117°C.
IR (cm⁻¹): 3332 (f, OH); 1251 (f, Ar-O).
¹H-NMR (CDCl₃ 200 MHz) δ (ppm): 1.61-2.03 (m, 2H, CH₂-CHOH); 2.29 (s, 3H, CH₃-C₅); 2.33 (s, 3H, CH₃-C₂); 2.56-2.80 (m, 6H, (CH₂)₃-N); 2.87 (t, 4H, (CH₂)₂-N-Ar); 4.86 (dd, 1H, CHOH); 5.30 (s.a., 2H, Alk-OH+Ar-OH); 6.65 (s, 1H, H₄); 6.76-7.19 (m, 4H, benzene).
EM-DIP (70 eV) m/z (% Abundance): 346(M⁺, 46); 191(100).

### Example 34: 1-(5-methylthiophen-2-yl)-3-[4-(2-methoxyphenyl)piperazin-1-yl]propan-1-ol (VN-2322) not part of the invention

M.p.: 142°C
IR (cm⁻¹): 3394 (f, OH); 1243 (f, Ar-O).
¹H-NMR (CDCl₃ 200 MHz) δ (ppm): 1.80-1.95 (m, 2H, CH₂-CHOH); 2.40 (s, 3H, CH₃); 2.95 (s, 6H, (CH₂)₃-N); 3.35 (s, 4H, (CH₂)₂-N-Ar); 3.77 (s, 3H, OCH₃); 4.78 (dd, 1H, CHOH); 5.68 (s.a., 1H, OH); 6.62 (d, 1H, H₄); 6.70 (d, 1H, H₃); 6.88-6.92 (m, 4H, benzene).
EM-DIP (70 eV) m/z (% Abundance): 346(M⁺; 100); 205(92)

### Example 35: 1-(3,5-dimethylbenzo[b]thiophen-2-yl)-3-[4-(2-methoxyphenyl)piperazin-1-yl] propan-1-ol (VN-7122)

M.p.: 79-80°C.
IR (KBr) (cm⁻¹): 3415 (f, OH); 1499 (m, C-N); 1240 (m, C-O).
¹H-NMR (DMSO- d₆, 200 MHz) δ: 1.77-1.97 (m, 2H, CHOH-CH₂); 2.29 (s, 3H, CH₃(C₅); 2.42 (s, 3H, CH₃(C₃); 2.44-2.52 (s.a., 6H, (CH₂)₃-N); 2.88-3.12 (s.a., 4H, (CH₂)₂-N-Ar; 3.75 (s, 3H, CH₃O); 5.12-5.20 (s.a., 1H, CHOH); 5.80-5.92 (s.a., 1H, OH); 6.85 (d, 4H, benzene); 7.15 (d, 1H, H₆); 7.45 (s, 1H, H₄); 7.7 (d, 1H, H₇).
EM-DIP (70 eV) m/z (% Abundance): 410(M⁺,76); 200(100); 148(16).

### Example 36: 1-(3-methylbenzo[b]thiophen-2-yl)-3-[4-(2-methoxyphenyl)piperazin-1-yl]propan-1-ol, (VN-7022)

M.p.: 145-147°C
IR (KBr) (cm⁻¹): 3405 (m, OH); 1498 (m, C-N).
¹H-NMR (DMSO- d₆, 200 MHz) δ: 1.82-1.95 (m, 2H, CHOH-CH₂); 2.33 (s, 3H, CH₃-Ar); 2.38-2.52 (s.a., 6H, (CH₂)₃-N); 2.90-3.05 (s.a., 4H, (CH₂)₂-N-Ar) 3.75 (s, 3H, CH₃O); 5:12-5.20 (s.a., 1H, CHOH); 5.91 (s, 1H, OH); 6.90 (d, 4H, benzene); 7.26-7.40 (m, 2H, H₆+H₅); 7.69(d, 1H, H₄); 7.88 (d, 1H, H₇).
EM-DIP (70 eV) m/z (% Abundance): 396(M⁺,68); 219(41); 205(100);134 (31).

### Example 37: 1-(3-methylbenzo[b]thiophen-2-yl)-3-[4-(2-hydroxyphenyl)piperazin-1-yl]propan-1-ol, (VN-702H)

M.p.: 149-151°C
IR (KBr) (cm⁻¹): 3398 (m, OH); 1490 (f, C-N).
¹H-NMR (DMSO-d₆, 200 MHz): 1.79-1.96 (m, 2H, CHOH-CH₂); 2.34 (s, 3H, CH₃); 2.43-2.57 (s.a, 6H, (CH₂)₃-N); 2.90-3.12 (s.a., 4H, (CH₂)₂-N-Ar); 5.19 (t, 1H, CHOH); 6.66-6.87 (m, 5H, benzene + OH); 7.27-7.41 (m, 2H, H₆+H₅); 7.70 (d, 1H, H₄); 7.90 (d, 1H, H₇).
EM-DIP (70 eV) m/z (% Abundance): 382(M⁺,77); 134(100); 120(79).

### Example 38: 1-(3-methylthiophen-2-yl)-3-[4-(2-methoxyphenyl)piperazin-1-yl]propan-1-ol (VN-2722) not part of the invention

M.p.: 91-93°C
IR (KBr) (cm⁻¹): 3413 (f, OH); 1594 (f, C-N).
¹H-NMR (CDCl₃, 200 MHz) δ: 1.81-2.00 (m, 2H, CHOH-CH₂); 2.18 (s, 3H, CH₃); 2.68-2.92 (m, 6H, (CH₂)₃-N); 3.11-3.14 (s.a., 4H, (CH₂)₂-N-Ar); 5.15-5.25 (m, 1H, CHOH); 6.76 (d, 1H, H₄); 6.87-7.05 (m, 4H, benzene); 7.11 (d, 1H, H₅).
EM-DIP (70 eV) m/z (% Abundance): 346(M⁺,85); 205(100); 150(65); 125(60).

### PROCESSS OF SYNTHESIS OF FORMULA (Ic) PRODUCTS

### Example 39: 1-(benzo[b]thiophen-3-yl)-3-[4-(2-methoxyphenyl)piperazin-1-yl]propan-1-one E-oxime. (VN-2282)

0.5 g of 1-(benzo[b]thiophen-3-yl)-3-[4-(2-methoxyphenyl)piperazin-1-yl]propan-1-one hydrochloride (1.20 x 10⁻³ moles) in 20 ml of EtOH and 0.5 g of hydroxylamine hydrochloride (7.19 x 10⁻³ moles) were refluxed for 1.5 hours. At the end of this time, the reaction was rendered basic with NaOH dissolved in EtOH and H₂O and was allowed to react 1 hour under reflux. H₂O was added to the medium, the excess EtOH was removed in the rotary evaporator and the aqueous phase was extracted with AcOEt. The organic phase was washed with H₂O, was dried over Na₂SO₄ and the solvent was removed. The product was purified with a silica column of using AcOEt/hexane as mobile phase. The E isomer of the oxime was prepared in this way. Yield: 60%.
M.p.: 191°C
IR (cm⁻¹): 3426 (f, OH); 1242 (mf, Ar-O)
¹H-NMR (CDCl₃ 200 MHz) δ (ppm): 2.60 (s.a., 6H, (CH₂)₃-N ); 2.95-3.16 (m, 6H, (CH₂)₂N, CH₂NOH); 3.77 (s, 3H, OCH₃) 6.87-6.92 (m, 4H, benzene); 7.40-7.43 (m, 2H, H₅+H₆); 8.01 (dd, 1H,H₇); 8.10 (s, 1H, H₂); 8.57 (dd, 1H, H₄); 11.31 (s, 1H, OH)
EM-DIP (70 eV) m/z (% Abundance): 395(M⁺; 4.1); 379(5.4); 205 (100)

### Example 40: 1-(2,5-dimethylthiophen-3-yl)-3-[4-(2-methoxyphenyl)piperazin-1-yl]propan-1-one Z-oxime. (VN-2582A) not part of the invention

A mixture of 1.02 g of 1-(2,5-dimethylthiophen-3-yl)-3-[4-(2-methoxyphenyl)piperazin-1-yl]propan-1-one hydrochloride (3.04 x 10⁻³ moles) and 1.2 g of hydroxylamine hydrochloride were heated with ethanol (30 ml) under reflux for 1.5 hours, after which the reaction was rendered basic with NaOH dissolved in EtOH-H₂O. The reaction was allowed to cool, was diluted with H₂O and the solvent was removed in the rotary evaporator. The product was obtained by silica gel column chromatography eluting with AcOEt/hexane (1:1).

### Z ISOMER

P. f.: 126°C.
IR (cm⁻¹):3350 (d, OH); 1594 (d, C=N); 1241 (f, Ar-O).
¹H-NMR (DMSO-d₆ 200 MHz) δ (ppm): 2.37-2.50 (m, 6H, (CH₂)₃-N); 2.40 (s, 3H, CH₃-C₅); 2.44 (s, 3H, CH₃-C₂); 2.79 (t, 2H, CH₂-CNOH); 2.93 (s, (CH₂)₂-N-Ar); 3.76 (s, 3H, OCH₃); 6.80 (s, 1H, H₃); 6.87-6.92 (m, 4H, benzene); 10.97 (s, 1H, OH).
EM-DIP (70 eV) m/z (% Abundance): 373(M⁺; 6.2); 205(100).

### Example 41: 1-(2,5-dimethylthiophen-3-yl)-3-[4-(2-methoxyphenyl)piperazin-1-yl]propan-1-one E-oxime, (VN-2582B) not part of the invention

Following the process described in the previous Example and eluting the silica gel column with AcOEt/hexane (1:1) a second fraction was obtained, containing the product corresponding to the E isomer.
E ISOMER
M.p.: 144°C.
IR (cm⁻¹):3278 (mf, OH); 1613 (f, C=N); 1266 (m, Ar-O).
¹H-NMR (DMSO-d₆ 200 MHz) δ (ppm): 2.08-2.44 (m, 6H, (CH₂)₃-N); 2.98 (t, 2H, CH₂-CNOH); 3.19-3.57 (m, (CH₂)₂-N-Ar); 3.80 (s, 3H, OCH₃); 6.63 (s, 1H, H₃); 6.91-7.00 (m, 4H, benzene); 11.53 (s, 1H, OH).
EM-DIP (70 eV) m/z (% Abundance): 373(M⁺; 7.5); 205(100).

### Example 42: 1-(3-methylthiophen-2-yl)-3-[4-(2-methoxyphenyl)piperazin-1-yl]propan-1-one Z-oxime (VN-2382A) not part of the invention

1 g of the product obtained in Example 13 was reacted in 25 ml of EtOH with 1 g of hydroxylamine hydrochloride for 1.5 hours after which the reaction was rendered basic with NaOH dissolved in EtOH and H₂O. Refluxing continued for a further half-hour. The reaction was cooled, H₂O was added, the excess EtOH was removed in the rotary evaporator and the residue was extracted with AcOEt. The organic phase was washed with water, was dried over Na₂SO₄. By silica gel column chromatography and eluting with CH₂Cl₂/MeOH (9:1), a first fraction was obtained, containing the product in the form of the Z isomer.
M.p.: 180-182°C.
IR (cm⁻¹): 1593 (d, C=N).
¹H-NMR (DMSO-d₆ 200 MHz) δ (ppm): 2.37 (s, 3H, CH₃); 2.65-2.73 (m, 6H, (CH₂)₃-N); 2.92 (dd; 2H, CH₂-CNOH); 3.06 (s, 4H, (CH₂)₂-N-Ar); 3.80 (s, 3H, OCH₃); 6.78 (d, 1H, H₄); 6.90-6.94 (m, 4H, benzene); 7.17 (d, 1H, H₃); 11.15 (s, 1H, OH).
EM-DIP (70 eV) m/z (% Abundance): 342(M⁺; 27); 205(100).

### Example 43: 1-(3-methylthiophen-2-yl)-3-[4-(2-methoxyphenyl)piperazin-1-yl]propan-1-one E-oxime. (VN-2382B) not part of the invention

Following the process described in the previous Example and eluting the chromatography column with CH₂Cl₂/MeOH (9:1), a second fraction was obtained, containing the product in the form of the E isomer.
M.p.: 171-173°C
IR (cm⁻¹): 1620 (d, C=N); 1246 (f, Ar-O).
¹H-NMR (DMSO-d₆ 200 MHz) δ (ppm): 2.48 (s, 3H, CH₃); 2.81-2.89 (m, 6H, (CH₂)₃-N); 2.98 (dd; 2H, CH₂-CNOH); 3.17 (s, 4H, (CH₂)₂-N-Ar); 3.86 (s, 3H, OCH₃); 6.91-6.95 (m, 4H, benzene + H₄); 7.38 (d, 1H, H₃); 11.56 (s, 1H, OH).
EM-DIP (70 eV) m/z (% Abundance): 342(M⁺; 52); 205(100).

Following a process similar to the one described in Example 41 and starting out from the corresponding ketone, the products listed below were prepared.

### Example 44: 3-[4-(2-methoxyphenyl)piperazin-1-yl]-1-(thiophen-3-yl)propan-1-one dihydrate dihydrochloride E,Z-oxime. (VN-2182) not part of the invention

M.p.: 144-146°C.
IR (cm⁻¹): 3416 (mf, OH); 1611 (d, C=N); 1268 (f, Ar-O).
¹H-NMR (DMSO-d₆ 200 MHz) δ (ppm): 3.12-3.26 (m, 6H, (CH₂)₃-N); 2.39-3.68 (m, 6H, CH₂CO, (CH₂)₂N-Ar); 6.95-7.00 (m, 4H, benzene); 7.45 (d; 1H, H₄); 7.60 (d, 1H, H₅); 8.16 (s, 1H, H₂); 11.50 (s, 0,5 H, OH Z isomer); 11.60 (s, 0,5H, OH E isomer).
EM-DIP (70 eV) m/z (% Abundance): 345(M+, 11); 328(27); 205(100).

### Example 45: 1-(3,5-dimethylbenzo[b]thiophen-2-yl)-3-[4-(2-methoxyphenyl)piperazin-1-yl]propan-1-one oxime.(VN-7182)

M.p.: 72-74°C.
IR (KBr) (cm⁻¹): 3421 (f, N-OH); 1450 (m, C-N).
¹H-NMR (DMSO-d₆, 200 MHz) δ: 2.42-2.63 (s.a., 14H, (CH₂)₃-N + 2CH₃[3+5] + CH₂CH₂NOH); 2.80-2.98 (s.a., 4H, CH₂)₂N); 3.73 (s, 3H, CH₃O-); 6.88 (d, 4H, benzene); 7.19 (d, 1H, H₆); 7.57 (s, 1H, H₄); 7.71-7.80 (m, 1H, H₇); 11.4-11.8 (s.a., 1H, OH).
EM-DIP (70 eV) m/z (% Abundance): 425(M⁺,2); 407(6); 205(100).

### Example 46: 1 (3-methylbenzo[b]thiophen-2-yl)-3-[4-(2-methoxyphenyl)piperazin-1-yl]propan-1-one oxime. (VN-7082)

M.p.: 82-84°C.
IR (KBr) (cm⁻¹): 3652 (p, N-OH); 1498 (f, C-N).
¹H-NMR (DMSO-d₆, 200 MHz) δ: 2.48-2.62 (s.a., 14H, (CH₂)₃-N-CH₃ + CH₂NOH ); 2.85- 3.03 (s.a., 4H, (CH₂)₂N); 3.77 (s, 3H, CH₃O); 6.85-7.02 (d, 4H, benzene); 7.35-7.50 (m, 2H, H₅+H₆); 7.79-7.85 (m, 1H, H₄); 7.90-7.99 (m, 1H, H₇); 11.52-11.82 (s.a., 1H, OH)
EM-DIP (70 eV) m/z (% Abundance): 409(M⁺, 13); 393(25); 205(100).

### PROCESS OF SYNTHESIS OF PRODUCTS OF FORMULA (Id)

### Example 47: 3-[4-(2-methoxyphenyl)piperazin-1-yl]-O-(n-propyl)-1-(thiophen-3-yl)propan-1-one oxime. (VN-218P2) not part of the invention

### 1. O-propylhydroxylamine

10 g of N-hydroxyphthalimide (60.0 x 10⁻³ moles) and 6.0 g of K₂CO₃ (40.0 x 10⁻³ moles) were stirred in an N-methyl-2-pyrrolidone medium (164 ml) in a 500 ml three-mouthed flask under an N₂ atmosphere, at room temperature, for 6 hours and thereafter 11.73 g of 1-bromopropane (57.0 x 10⁻³ moles) were added. After the addition, the temperature was raised to 50°C and held at this level for 15 hours, the reaction mass was cooled and a precipitate was collected, was washed with cold EtOH. In this way, the N-(O-propyl)phthalimide was obtained, which was refluxed under EtOH (85 ml) with 2.16 ml of hydrazine hydrate (43 x 10⁻³ moles) for 30 minutes. The reaction was checked by TLC until the starting product was no longer visible. The mass was cooled and H₂O was added, whereby the O-propylhydroxylamine precipitated out. Yield: 75%.

### 2. 3-[4-(2-methoxyphenyl)piperazin-1-yl]-O-(n-propyl)-1-(thiophen-3-yl)propan-1-one oxime

0.5g of 3-[4-(2- methoxyphenyl)piperazin-1-yl]-1-(thiophen-3-yl)propan-1-one (1.36 x 10⁻³ moles) and 0.10 g of O-propylhydroxylamine (1.36 x 10⁻³ moles) were refluxed for 1.5 hours in 15 ml of EtOH after which the medium was rendered basic with NaOH dissolved in EtOH and H₂O. The reflux was maintained and the appearance of a new product was observed in TLC. After cooling the reaction and adding H₂O, the excess EtOH was removed in the rotary evaporator. The resulting aqueous phase was extracted with AcOEt and this was washed with H₂O, and a saturated solution of NaCl, was dried over Na₂SO₄ and the solvent was removed. The residue was introduced into a silica column using AcOEt/hexane as mobile phase. The product was extracted therefrom in the form of oil. Yield: 22%.
IR (cm⁻¹): 1594 (f, C=N); 1240 (f, Ar-O).
¹H-NMR (CDCl₃ 200 MHz) δ (ppm): 0.90 (t, 3H, CH₂-CH₃); 1.65 (m, 2H, CH₂-CH₃); 2.57-2.89 (m, 6H, (CH₂)₂-N);2.86-2.94 (m, 2H, CNOH-CH₂); 3.05 (s, 4H, (CH₂)₂N-Ar); 3.79 (s, 3H, OCH₃); 4.04 (t, 2H, O-CH₂-CH₃); 6.76-6.96 (m, 4H, benzene); 7.19-7.22 (m, 1H, H₄); 7.37-7.42 (m, 2H, H₂ + H₅).
EM-DIP (70 eV) m/z (% Abundance): 387(M⁺, 4.3); 205(100).

### Example 48: O-ethyl-3-[4-(2-methoxyphenyl)piperazin-1-yl]-1-(thiophenyl)propan-1-one oxime. (VN-218E2) not part of the invention

Following the process described in the previous Example, starting from the product obtained in Example 7 and reacting it with O-ethyl hydroxylamine, the desired product is obtained.
IR (cm⁻¹): 1612 (d, C=O); 1254 (f, Ar-O).
¹H-NMR (DMSO-d₆ 200 MHz) δ (ppm): 1.31 (t, 3H, CH₂-CH₃); 2.62-2.65 (m, 6H, (CH₂)₃-N); 2.92-3.00 (m, 2H, CH₂C=N-O); 3.12 (s, 4H, (CH₂)₂N-Ar); 3.86 (s, 3H, OCH₃); 4.21 (c, 2H, O-CH₂-CH₃); 6.84-7.00 (m, 4H, benzene); 7.26-7.31 (m, 1H, H₄); 7.42-7.48 (m, 2H, H₂+H₅).
EM-DIP (70 eV) m/z (% Abundance): 373(M⁺; 7.7); 328(1.2); 205 (100).

### PROCESSES OF SYNTHESIS OF PRODUCTS OF FORMULA (Ie)

### Method A

### Example 49: 3-[4-(2-methoxyphenyl)piperazin-1-yl]-1-(thiophen-3-yl)-1-(1-naphthyloxy)propane hydrochloride. (VN-2142) not part of the invention

A mixture of 0.8 g of 3-[4-(2-methoxyphenyl)piperazin-1-yl]-1-(thiophen-3-yl)propan-1-ol, prepared as described in Example 29, and 0.06 g of NaH (2.4 x 10⁻³ moles) in 20 ml of dimethylacetamide was heated under stirring at 70°C for 1.5 hours until the salt was formed. At the end of this time, 0.35 g, 0.26 ml, of 1-fluoronaphthalene (2.4 x 10⁻³ moles) were added. The reaction was held at 105°C for 8 hours. After this time the reaction was allowed to cool. H₂O was added and it was extracted with ether, the organic phases being washed with H₂O. The solvent was removed to dryness and an oily residue was obtained which was purified in silica column using AcOEt/hexane (1:1) as mobile phase. The hydrochloride was precipitated in acetone/HCl(c). Yield: 50%.
M.p.: 86 - 88°C.
IR (cm⁻¹): 1239 (mf, C-O).
¹H-NMR (DMSO-d₆ 200 MHz) δ (ppm): 2.59-2.66 (m, 2H, CH₂-C); 3.04-3.60 (m, 10H, CH₂); 5.81-5.86 (dd, 1H, CH); 6.92-7.08 (m, 4H, benzene); 7.19-7.55 (m, 7H, naphthalene); 7.58 (s; 1H, H₂); 7.83-7.86 (dd, 1H, H₄); 8.33-8.36 (dd, 1H, H₅).
EM-DIP (70 eV) m/z (% Abundance): 458(M⁺; 4); 205(100); 190(15).

### Example 50: 3-[4-(2-methoxyphenyl)piperazin-1-yl]-1-(thiophen-3-yl)-1-(p-trifluoromethylphenoxy)propane hydrochloride. (VN-2132) not part of the invention

Following the process described in the previous Example and using p-fluorobenzotrifluoromethyl in stead of p-fluoronaphthalene, the desired product was obtained, which was precipitated in the form of hydrochloride with acetone/HCl.
M.p.: 143-144°C
IR (cm⁻¹): 1251 (f, C-O).
¹H-NMR (DMSO-d₆ 200 MHz) δ (ppm): 2.49 (m, 2H, CH₂,C); 3.09-3.60 (m, 10H, CH₂); 5.72-5.79 (dd, 1H, CH); 6.89-7.02 (m, 4H, benzene); 7.12-7.19 (m, 3H, thiophene); 7.55-7.63 (m, 4H, p-substitution).
EM-DIP (70 eV) m/z (% Abundance): 476(M⁺; 25); 219(28); 205(100); 190(32).

### Example 51: 3-[4-(2-methoxyphenyl)piperazin-1-yl]-1-(thiophen-2-yl)-1-(1-naphthyloxy)propane dihydrochloride. (VN-2042) not part of the invention

Following the process described in Example 49 and using as starting product the compound obtained in Example 31 the desired product was obtained.
IR (cm⁻¹): 1244 (mf, C-O).
¹H-NMR (DMSO-d₆ 200 MHz) δ (ppm): 2.71 (m, 2H, CH₂-C); 3.14 (m, 6H, (CH₂)₃-N); 3.37-3.53 (m, 4H, (CH₂)₂-N-Ar); 5.08 (dd, 1H, CHOH); 6.92-7.56 (m, 13H, 7H naphthalene + 4H benzene 2H thiophene); 8.16-8.20 (dd, 1H, H₅).
EM-DIP (70 eV) m/z (% Abundance): 458(M⁺; 5); 205(18); 190(12).

### Method B

### Example 52: 3-[4-(2-methoxyphenyl)piperazin-1-yl]-1-(thiophen-3-yl)-1-(3,4-methylenedioxyphenoxy)propane (VN-2172) not part of the invention

To a suspension cooled to 0°C of 1.6 g of 3-[4-(2-methoxyphenyl)piperazin-1-yl]-1-(thiophen-3-yl)-propan-1-ol (3.6x10⁻³ moles), prepared as described in Example 29, and 1.7 g K₂CO₃ (12.10⁻³ ml) in 20 ml of dry acetone, were added dropwise 1.4 g, (1 ml) of mesyl chloride (12 x 10⁻³ moles) with stirring'over a period of 10 minutes. The mixture was held under stirring for 24 hours at a temperature ranging from 0 to 4°C, in the absence of light. After this time, H₂O was added and the mass was extracted with chloroform. The organic phases were washed with H₂O and were dried over Na₂SO₄. The solvent was removed to dryness and an oil was obtained corresponding to the mesylated derivative and was used directly in the following reaction without being identified. To a solution of 0.11 g of NaOH (2.82 x 10⁻³ moles) in 7 ml of EtOH were added a room temperature 0.52 g of 3,4-methylenedioxyphenol (3.8 x 10⁻³ moles). After 10 minutes, a solution of 1.2 g of the mesylated derivative (2.82 x 10⁻³ moles) dissolved in 7 ml of EtOH was added dropwise. The mixture was left under stirring at room temperature for 4 days. After this time, water was added and it was extracted with chloroform. The organic phases were washed with H₂O and were dried over Na₂SO₄. The solvent was removed by concentration to dryness and an oily residue was obtained, which was purified in silica column with AcOEt/hexane (1:1) as mobile phase. Yield: 2%.
M.p.: 175-177°C.
IR (cm⁻¹): 1239 (f, C-O).
¹H-NMR (CDCl₃ 200 MHz) δ (ppm): 2.44-2.55 (m, 2H, CH₂-CHOH); 2.79-2.91 (m, 6H, (CH₂)₃-N; 3.44-3.54 (m, 4H, (CH₂)₂-N-Ar); 5.83-5.92 (m, 3H, CHOH+-CH₂-O); 6.44-6.53 (m, 3H, 3,4-methylenedioxyphenoxy); 6.86-7.27 (m, 7H, 4H benzene+3H thiophene).
EM-DIP (70 eV) m/z (% Abundance): 205(79); 136(54).

### PROCESSES OF SYNTHESIS OF PRODUCTS OF FORMULA (If)

### Example 53: 1-(2,5-dimethylthiophen-3-yl)-3-[4-(2-methoxyphenyl)piperazin-1-yl]-1-propane N-isopropylcarbamate (VN-2552) not part of the invention

1.67 g of 1-(2,5-dimethylthiophen-3-yl)-3-[4-(2-methoxyphenyl)piperazin-1-yl]propan-1-ol (3.24 x 10⁻³ moles), prepared as described in Example 32, were reacted in 20 ml of dry dioxane at room temperature with 0.5 ml of isopropylisocyanate for 4 days (on the second day a further 0.5 ml of isocyanate was added). The reaction was followed by TLC. H₂O was added to the reaction mass, it was extracted with AcOEt, the organic phase was washed with H₂O, was dried over Na₂SO₄ and the solvent was removed. The product was purified in a silica column using AcOEt/hexane as mobile phase. The desired product was obtained in the form of an oil. Yield: 10%.
IR (cm⁻¹): 3333 (m, NH); 1713 (f, C=O); 1240 (mf, Ar-O).
¹H-NMR (DMSO-d₆ 200 MHz) δ (ppm): 1.06 (d, 6H, (CH₃)₂-CH); 1.79-2.17 (m, 2H, CH₂-CHO-R); 2.32 (s, 3H, CH₃-C₅); 2.35 (s, 3H, CH₃-C₂); 2.56 (m, 6H, (CH₂)₃-N); 3.03 (t, 4H, (CH₂)₂-N-Ar); 3.66-3.79 (m, 1H, CH-(Me)₂); 3.79 (s, 3H, OCH₃); 5.70 (dd, 1H, CHOR); 6.53 (s, 1H, H₃); 6.77-6.93 (m, 4H, benzene).
EM-DIP (70 eV) m/z (% Abundance): 445(M⁺, 3.5); 359(3); 205(100).

### Example 54: 1-(2-methoxyphenyl)-4-[3-thiophen-3-yl-3-(3-trifluoromethylphenoxy)-propyl]piperazine. (VN-2152) not part of the invention

Following the process described in the previous Example, but starting out from the product obtained in Example 29, the desired product was obtained.
M.p.: 98-99°C.
IR (cm⁻¹): 3349 (mf, NH); 1688 (mf, C=O); 1263 (mf, Ar-O).
¹H-NMR (CDCl₃ 200 MHz) δ (ppm): 1.12 (d, 6H, (CH₃)₂-CH); 1.97-2.22 (m, 2H, CH₂-CHO-R); 2.41 (t, 2H, CHORCH₂-CH₂-N); 2.63 (m, 4H, (CH₂)₂-N); 3.07 (t, 4H, (CH₂)₂-N-Ar); 3.73-3.83 (m, 1H, CH-(CH₃)₂; 3.83 (s, 3H, OCH₃); 5.84 (dd, 1H, CHOR); 6.81-6.98 (m, 4H, benzene); 7.06 (d, 1H, H₄); 7.22-7.29 (m, 2H, H₂, H₅).
EM-DIP (70 eV) m/z (% Abundance): 417(M+, 22); 331(23); 205(100).

The results obtained in the pharmacological valuation tests for the products of the present invention are summarised in Tables 1,2,3,4.

### Example 55: Enantiomeric resolution of the product 1-(benzo[b]thiophen-3-yl)-3-[4-(2-methoxyphenyl)piperazin-1-yl]-propan-1-ol. (VN-2222)

Chloroform (2 ml), triethylamine (538 µl, 3.88 mmoles) and 4-dimethylaminopyridine (108 mg, 0.88 mmoles) were added to each of two test tubes containing 500 mg (1.31 mmoles) of the racemic mixture of VN-2222. These solutions were added over two flasks containing respectively 531 mg (2.88 mmoles) and 542 mg (2.94 mmoles) of (S)-(+)-α-methoxyphenylacetic acid chloride. They were allowed to react for one hour and were pooled into one flask, with the addition of chloroform up to 50 ml. The mixture was washed with: dilute HCl (50 ml x 3), dilute Na₂CO₃ (50 ml x 3), saturated NaCl (50 ml x 2), H₂O (50 ml x 2). The chloroform was removed at reduced pressure, giving an oil corresponding to the mixture of diastereomers (R)-VN-2222-(S)-OMM and (S)-VN-2222(S)-OMM (880 mg, 1.66 mmoles).

HPLC [HPLC Waters 600E; LED detector Waters 994; work station Millennium; Supelcosil LC-CN column, 25 x 0.46 cm; mobile phase: (hexane/isopropanol+triethylamine, 90/10); flow rate: 0.7 ml/min]: dwell time (tᵣ) in minutes; 8.2 diastereomer (R)-VN-2222-(S)-OMM and 9.3 diastereomer (S)-VN-2222-(S)-OMM.

Thereafter, the mixture of diastereomers dissolved in ethyl acetate was separated by preparative TLC using 20 x 40 cm silica gel 60 plates. Mobile phase: TDA (Toluene, dioxane, acetic acid 90:25:4). Two bands appear at 8 cm (S, S) and 12 cm (R,S) (UV: 254 nm). Ethyl acetate was added to the silica corresponding to each diastereomer, followed by filtration and the solvent was removed at reduced pressure. Both diastereomers were obtained separately: 420 mg (0.79 mmoles) of (R,S) and 375 mg (0.71 mmoles) of (S,S).
(R)-VN-2222-(S)-OMM:
¹H-NMR(CDCl₃, 200 MHz) 8 (ppm): 2.06-2.23 (m, 4H, CHOH-CH₂ + CH₂-N); 2.44 (m, 4H, (CH₂)₂-N); 3.02 (m, 4H, (CH₂)₂N-Ar); 3.37 (s, 3H, CH₃O-OMM); 3.83 (s, 3H, CH₃O); 4.77 (s, 1H, OMM); 6.36 (t, 1H, CHOH-OMM); 6.81-6.99 (m, 4H, benzene); 7.33-7.51 (m, 8H, H₂ + H₅ + H₆ + 5H benzene OMM); 7.69-7.89 (m, 2H, H₄ + H₇).
HPLC: tᵣ: 8.2 min.
(S)-VN-2222-(S)-OMM:
¹H-NMR(CDCl₃, 200 MHz) δ (ppm): 2.23-2.26 (m, 2H, CHOH-CH₂); 2.38-2.43 (m, 2H, CH₂-N); 2.59 (m, 4H, (CH₂)₂-N); 3.07 (m, 4H, (CH₂)₂N-Ar); 3.38 (s, 3H, CH₃O-OMM); 3.84 (s, 3H, CH₃O); 4.83 (s, 1H, OMM); 6.34 (t, 1H, CHOH-OMM); 6.83-6.99 (m, 5H, benzene + H₂); 7.24-7.40 (m, 7H, H₅ + H₆ + 5H benzene OMM); 7.69-7.80 (m, 2H, H₄ + H₇).
HPLC: tᵣ: 9.3 min

The next step was hydrolysis under non-racemising conditions. Each of the diastereomers was dissolved in methanol (40 ml), an excess of K₂CO₃ was added, and the reaction mass was held at room temperature under constant stirring for 5 hours. The K₂CO₃ was removed by filtration, the solvent was removed, water was added and extractions in chloroform (3 x 50 ml) were carried out. The chloroform was removed at reduced pressure, yielding 172 mg (0.45 mmoles) of the enantiomer (R) and 97 mg (0.25 mmoles) of the enantiomer (S). Both enantiomers have the same ¹H-NMR spectrum.
VN-2222: ¹H-NMR(CDCl₃, 200 MHz) δ (ppm): 2.09 (C, 2H, CHOH-CH₂); 2.6-2.9 (m, 6H, (CH₂)₃N); 3.1-3.3 (m, 4H, (CH₂)₂N-Ar); 3.86 (s, 3H, OCH₃); 5.35 (t, 1H, CHOH); 7.01-7.31 (m, 4H, benzene); 7.4 (m, 2H, H₅ + H₆); 7.44 (d, 1H, H₂); 7.78-7.789 (m, 2H, H₄ + H₇)
HPLC: tᵣ: 12 min.

To determine the enantiomeric purity, each enantiomer was derived with (R)-(+)-α-methoxy-α-(trifluoromethyl)phenylacetic acid chloride. Two vials were prepared with 5 mg (0.013 mmoles) of each enantiomer, chloroform (2 ml), triethylamine (6 µl, 0.039 mmoles) and 4-dimethylaminopyridine (2 mg, 0.016 mmoles). Two solutions were obtained, which were added over each of the flasks containing the (R)-(+)-α-methoxy-α-(trifluoromethyl)phenylacetic acid chloride (2 ml of hexane, N, N-dimethylformamide (4 µl, 0,05 mmoles) and oxalyl chloride (19 µl, 0,20 mmoles) were added to two vials containing (R)-(+)-α-methoxy-α-(trifluoromethyl)phenylacetic acid (10 mg, 0,043 mmoles), were left to react for one hour, were filtered and the solvent was removed, yielding the chloride of the acid, 7,1 mg (0.03 mmoles) and 7.0 mg (0.03 mmoles), respectively). Both flasks were allowed to rest for one hour, followed by the addition of 10 ml of chloroform. They were washed with: dilute HCl (10 ml x 3), dilute Na₂CO₃ (10 ml x 3), saturated NaCl (10 ml x 2), H₂O (10 ml x 2). The chloroform was removed, yielding in each case an oil corresponding to each of the diastereomers: (1R)-1-(benzo[b]thiophen-3-yl)-3-[4-(2-methoxyphenyl)piperazin-1-yl]propyl-(2R)-3,3,3-trifluoro-2-methoxy-2-phenylacetate, 4.2 mg (0.007 mmoles), and (1S)-1-(benzo[b]thiophen-3-yl)-3-[4-(2-methoxyphenyl)piperazin-1-yl]propyl-(2R)-3,3,3-trifluoro-2-methoxy-2-phenylacetate, 4 mg (0.007 mmoles).
(R)-VN-2222-(R)-MTPA:
¹H-NMR(CDCl₃, 200 MHz) δ (ppm): 2.10-2.48 (m, 4H, CHOH-CH₂ + CH₂-N); 2.52 (m, 4H, (CH₂)₂-N); 3.01 (m, 4H, (CH₂)₂-N-Ar); 3.47 (c, 3H, CH₃O-MTPA); 3.84(s, 3H, CH₃O); 6.49 (t, 1H, CHOH-MTPA); 6.77-7.01 (m, 4H, benzene); 7.15-7.37 (m, 8H, H₂+ H₅ + H₆ + 5H benzene MTPA); 7.73-7.82 (m, 2H, H₄ + H₇).
HPLC: tᵣ: 6.3 min.
(S)-VN-2222-(R)-MTPA:
¹H-NMR(CDCl₃, 200 MHz) δ (ppm): 2.10-2.48 (m, 4H, CHOH-CH₂ + CH₂-N); 2.52 (m, 4H, (CH₂)₂-N); 3.01 (m, 4H, (CH₂)₂-N-Ar); 3.35 (c, 3H, CH₃O-MTPA); 3.84 (s, 3H, CH₃O); 6.44 (t, 1H, CHOH-MTPA); 6.77-7.01 (m, 4H, benzene); 7.15-7.37 (m, 8H, H₂+H₅+H₆ + 5H benzene MTPA); 7.73-7.82 (m, 2H, H₄ + H₇).
HPLC: tᵣ: 6.3 min.

### DESCRIPTION OF THE METHODS USED TO EVALUATE THE PHARMACOLOGICAL PROPERTIES

Some examples of benzothiophenes and thiophenes are described below. Although the thiophenes are not object of the invention, they have been included on a comparative basis.

### Test for binding to 5HT_{1A} receptors

To determine the affinity of the products to the 5HT_{1A} receptors, binding tests were carried out, using as radioligand the agonist ³H-dipropylaminotetraline (DPAT) following the technique described by Hoyer et al. (Eur. J. Pharmacol., 118, 13-23) (1985).

Rat front cortex was dried and homogenised in Tris-HCl 50 mM pH 7.7 at 4°C. The resulting homogenate was centrifuged at 25.000 r.p.m. for 15 min. and the pellet obtained was resuspended in Tris-HCl and incubated at 37°C for 10 min. The resulting resuspension was centrifuged again and resuspended in Tris-HCl containing 4 mM of CaCl₂ 4 mM. For the binding test, the incubation mixture contained the membrane suspension, ³H-DPAT (1 nM) and the cold displacer. Rapid filtration was carried out to separate the fraction bound to the receptors.

### Binding tests to the 5-HT carrier

The rat front cortex membrane fraction was prepared as described for the determination of the binding to the 5-HT_{1A} receptors. The membrane suspension was incubated for 60 min at 22°C with ³H-paroxetine using fluoxetine as displacer. On completion of the incubation, the membrane fraction was separated by rapid filtration. The technique used is the one described by Marcusson et al. (J. Neurochemistry, 44, 705-711) (1985).

The results obtained in these pharmacological evaluation tests for the products of the present invention are summarised in Tables 1, 2, 3, 4.

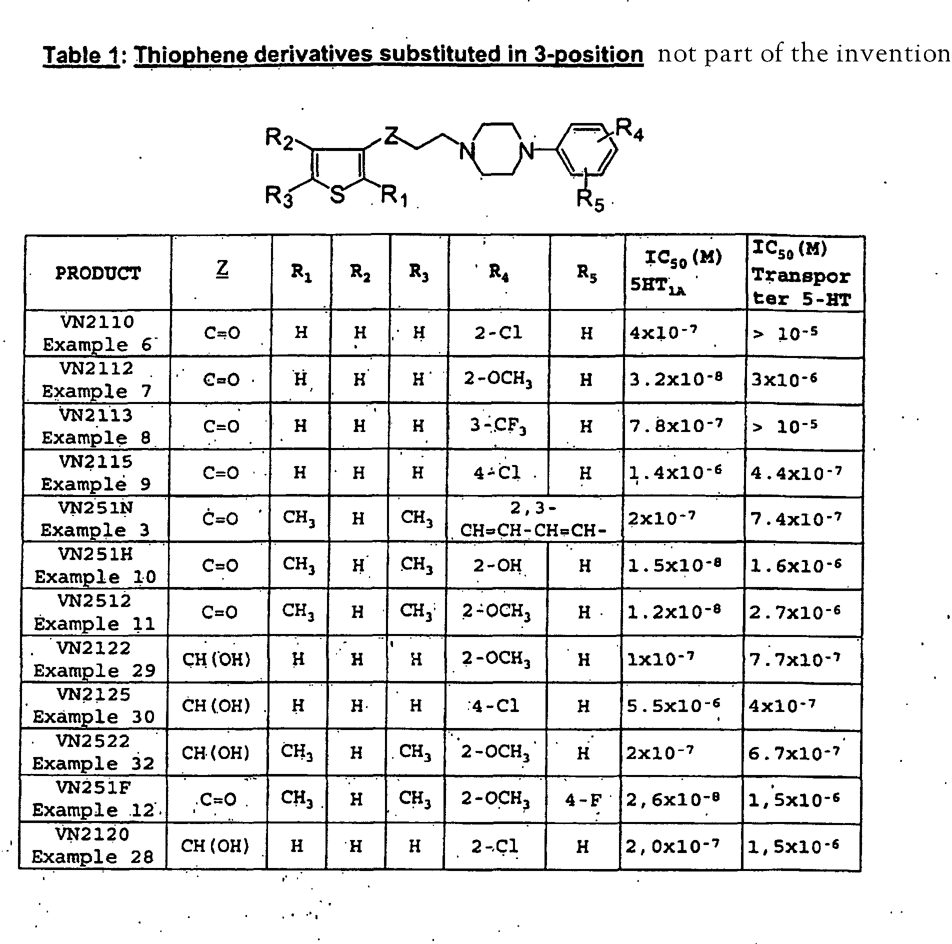

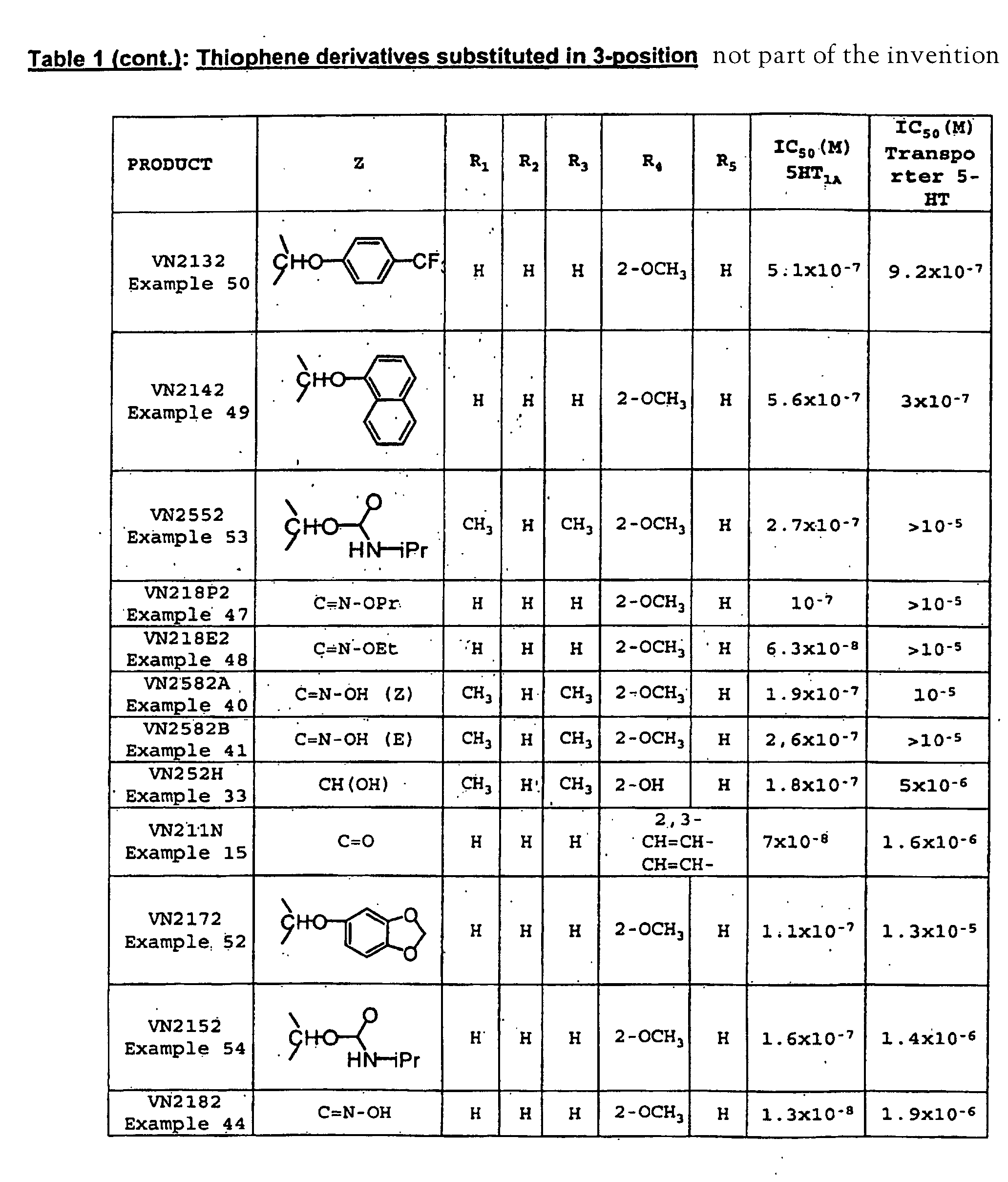

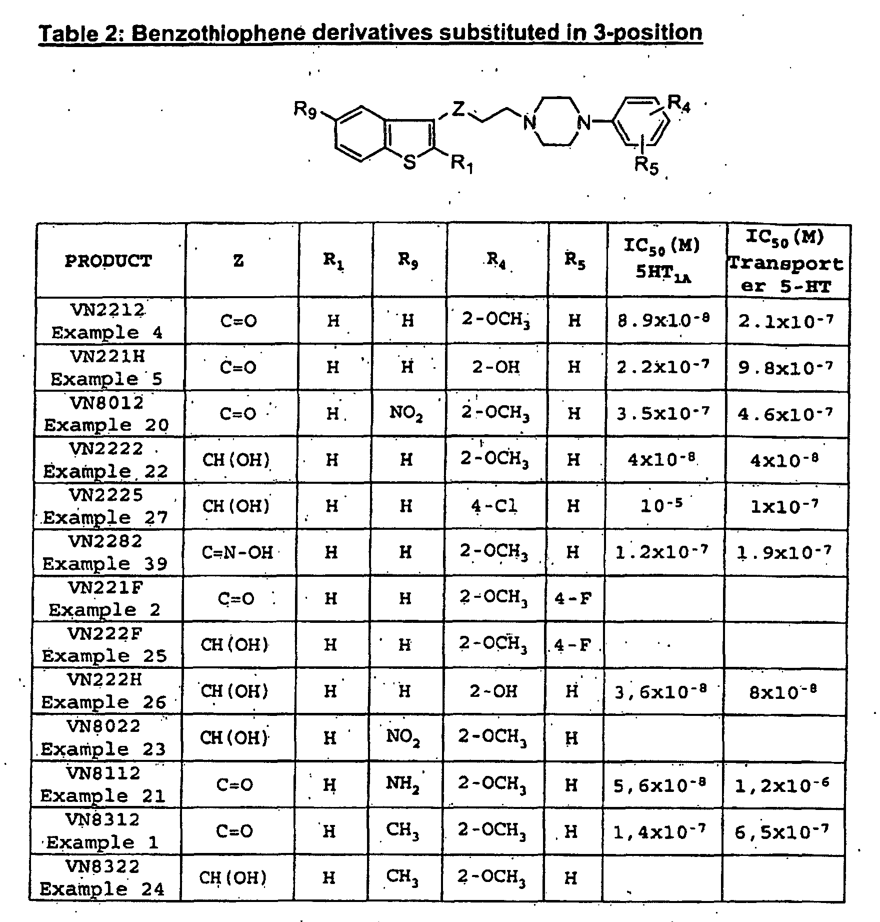

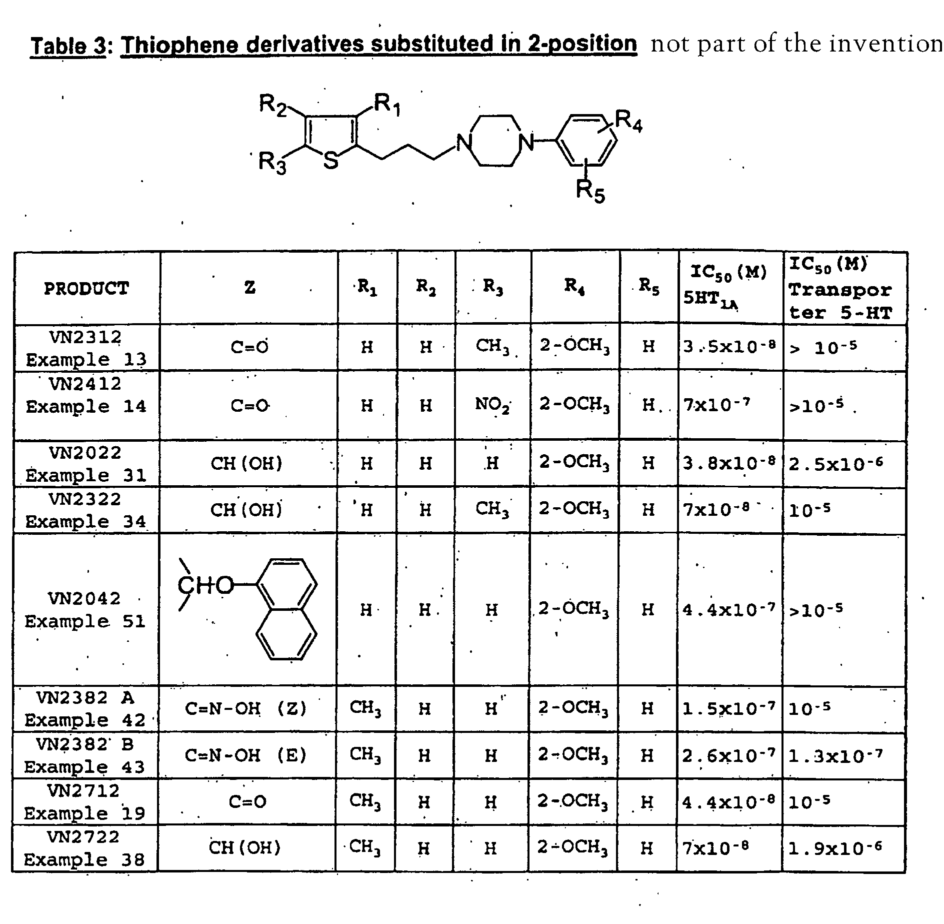

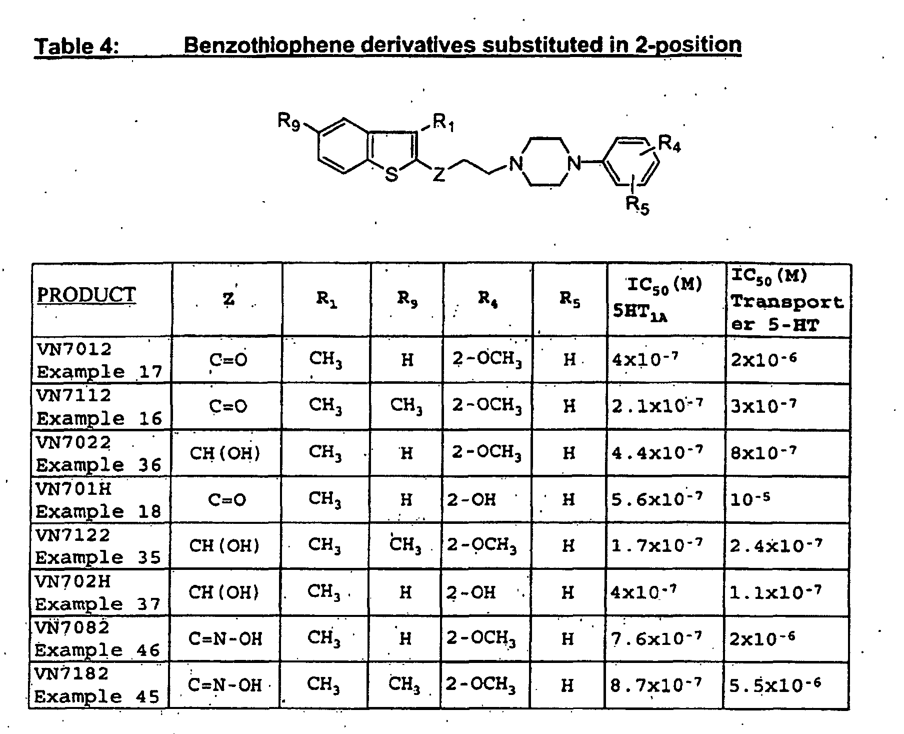

## Claims

1. Benzothiophene derivative compounds of the general formula (I): 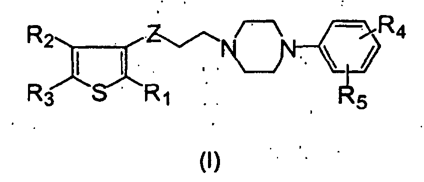 where:
Z is: -CO-, -CH(OR₆)-, -C(NOR₇)-;
R₁ is: H, C₁-C₆ alkyl, halogen, or -OR₆;
R₂ and R_{3'} together are: -C(R₈) = C(R₉)-C(R₁₀)=C(R₁₁)-;
R₄ and R₅ are the same or different and stand for: H, C₁-C₆ alkyl, halogen, haloalkyl, -OR₁₂, nitro, NR₁₃R₁₄; COR₁₂; CO₂R₁₂; -SO₂NR₁₃R₁₄; -SO₂R₁₂; SR₁₂, cyano; -CONR₁₃R₁₄ or R₄ and R₅ together form a benzene ring fused to the phenyl ring;
R₆ is: H, C₁-C₆ alkyl, CO₂R₁₂, -C(O)NR₁₃R₁₄, naphthyl or phenyl optionally substituted by one or more substituents selected from among: H, C₁-C₆ haloalkyl, C₁-C₆ alkyl, halogen, C₁-C₆ alkoxy, methylenedioxy, nitro, cyano;
R₇ is: H or C₁-C₆ alkyl;
R₈, R₉, R₁₀ and R₁₁ are independent and stand for: H, C₁-C₆ alkyl, halogen, -OR₁₂, nitro, cyano, NR₁₃R₁₄; -COR₁₂; CO₂R₁₂; -SO₂NR₁₃R₁₄; -SO₂R₁₂; SR₁₂, -CONR₁₃R₁₄;
R₁₂ is H, C₁-C₆ alkyl or phenyl;
and R₁₃ and R₁₄ are independent and stand for: H, C₁-C₆ alkyl or phenyl or R₁₃ and R₁₄, together with the atom of N to which they are attached, form a 5- or 6- membered ring in which there may optionally be an atom of N, O or S;
or a salt, or a solvate, or a geometric isomer, or an optical isomer, or a polymorph thereof.

2. The compounds of claim 1, where Z is -CO-:
1-(benzo[b]thiophen-3-yl)-3-[4-(2-methoxyphenyl)piperazin-1-yl]propan-1-one
1-(3,5-dimethylbenzo[b]thiophen-2-yl)-3-[4-(2-methoxyphenyl)piperazin-1-yl]propan-1-one

3. The compounds of claim 1, where Z is -CH(OR₆)-:
1-(benzo[b]thiophen-3-yl)-3-[4-(2-methoxyphenyl)piperazin-1-yl]propan-1-ol
1-(benzo[b]thiophen-3-yl)-3-[4-(2-hydroxyphenyl)piperazin-1-yl]propan-1-ol
1-(3,5-dimethylbenzo[b]thiophen-2-yl)-3-[4-(2-methoxyphenyl)piperazin-1-yl]propan-1-ol
1-(3-methylbenzo[b]thiophen-2-yl)-3-[4-(2-hydroxyphenyl)piperazin-1-yl]propan-1-ol

4. The compounds of claim 1, where Z is -C(NOR₇)-:
1-(benzo[b]thiophen-3-yl)-3-[4-(2-methoxyphenyl)piperazin-1-yl]propan-1-one oxime

5. The use of a compound according to any one of claims 1 to 4 for the preparation of a pharmaceutical composition for the treatment of neurological disorders.

6. The use of claim 5, for the preparation of a pharmaceutical composition for the treatment of anxiety and/or depression.

7. A pharmaceutical composition comprising a compound according to one of claims 1 to 4, in a therapeutically active amount for the treatment of anxiety and/or depression and an adequate amount of a carrier.

## Patentansprüche

1. Benzothiophenderivatverbindungen mit der allgemeinen Formel (I): 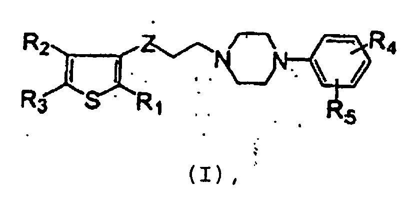 wobei
Z -CO-, -CH(OR₆)-, -C(NOR₇)- ist;
R₁ H, C₁-C₆-Alkyl, Halogen oder -OR₆ ist;
R₂ und R₃ zusammen -C(R₈)=C(R₉)-C(R₁₀)=C(R₁₁)- sind;
R₄ und R₅ gleich oder verschieden sind und für H, C₁-C₆-Alkyl, Halogen, Halogenalkyl, -OR₁₂, Nitro, NR₁₃R₁₄, COR₁₂, CO₂R₁₂, -SO₂NR₁₃R₁₄, -SO₂R₁₂, SR₁₂, Cyano, -CONR₁₃R₁₄ stehen oder R₄ und R₅ zusammen einen Benzolring bilden, der an dem Phenylring kondensiert ist;
R₆ H, C₁-C₆-Alkyl, CO₂R₁₂, -C(O)NR₁₃R₁₄, Naphthyl oder Phenyl ist, wahlweise mit einem oder mehreren Substituenten substituiert, der (die) ausgewählt ist (sind) aus H, C₁-C₆-Halogenalkyl, C₁-C₆-Alkyl, Halogen, C₁-C₆-Alkoxy, Methylendioxy, Nitro, Cyano;
R₇ H oder C₁-C₆-Alkyl ist;
R₈, R₉, R₁₀ und R₁₁ unabhängig sind und für H, C₁-C₆-Alkyl, Halogen, -OR₁₂, Nitro, Cyano, NR₁₃R₁₄, -COR₁₂, CO₂R₁₂, -SO₂NR₁₃R₁₄, -SO₂R₁₂, SR₁₂, -CONR₁₃R₁₄ stehen;
R₁₂ H, C₁-C₆-Alkyl oder Phenyl ist;
und R₁₃ und R₁₄ unabhängig sind und für H, C₁-C₆-Alkyl oder Phenyl stehen oder R₁₃ und R₁₄ zusammen mit dem N-Atom, an das sie gebunden sind, einen 5- oder 6-gliedrigen Ring bilden, in dem wahlweise ein N-, O-oder S-Atom vorhanden sein kann;
oder ein Salz oder ein Solvat oder ein geometrisches Isomer oder ein optisches Isomer oder ein Polymorph davon.

2. Verbindungen nach Anspruch 1, wobei Z -CO- ist:
1-(Benzo[b]thiophen-3-yl)-3-[4-(2-methoxyphenyl)piperazin-1-yl]propan-1-on
1-(3,5-Dimethylbenzo[b]thiophen-2-yl)-3-[4-(2-methoxyphenyl)piperazin-1-yl]propan-1-on

3. Verbindungen nach Anspruch 1, wobei Z -CH(OR₆)- ist:
1-(Benzo[b]thiophen-3-yl)-3-[4-(2-methoxyphenyl)piperazin-1-yl]propan-1-ol
1-(Benzo[b]thiophen-3-yl)-3-[4-(2-hydroxyphenyl)piperazin-1-yl]propan-1-ol
1-(3,5-Dimethylbenzo[b]thiophen-2-yl)-3-[4-(2-methoxyphenyl)piperazin-1-yl]propan-1-ol
1-(3-Methylbenzo[b]thiophen-2-yl)-3-[4-(2-hydroxyphenyl)piperazin-1-yl]propan-1-ol

4. Verbindungen nach Anspruch 1, wobei Z -C(NOR₇)- ist:
1-(Benzo[b]thiophen-3-yl)-3-[4-(2-methoxyphenyl)piperazin-1-yl]propan-1-onoxim

5. Benutzung einer Verbindung nach einem der Ansprüche 1 bis 4 zur Herstellung einer pharmazeutischen Zusammensetzung zur Behandlung von neurologischen Störungen.

6. Benutzung von Anspruch 5 zur Herstellung einer pharmazeutischen Zusammensetzung zur Behandlung von Angst und/oder Depression.

7. Pharmazeutische Zusammensetzung, die eine Verbindung nach einem der Ansprüche 1 bis 4 zur Behandlung von Angst und/oder Depression in einer therapeutisch aktiven Menge und eine angemessene Menge eines Trägers umfaßt.

## Revendications

1. Composés dérivés du benzothiophène de formule générale (I) : 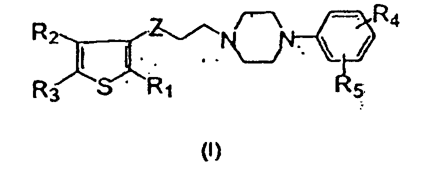 où
Z est : -CO-, -CH(OR₆)-, -C(NOR₇)- ;
R₁ est : H, un groupe alkyle en C₁-C₆, un halogène, ou -OR₆ ;
R₂ et R₃ ensemble sont : -C(R₈)=C(R₉)-C(R₁₀)=C(R₁₁)- ;
R₄ et R₅ sont identiques ou différents et représentent : H, un groupe alkyle en C₁-C₆, un halogène, un groupe halogénoalkyle, -OR₁₂, nitro, NR₁₃R₁₄ ; COR₁₂ : CO₂R₁₂ ; -SO₂NR₁₃R₁₄ ; -SO₂R₁₂ ; SR₁₂, cyano ; -CONR₁₃R₁₄ ou R₄ et R₅ forment ensemble un noyau benzénique condensé avec le noyau phényle ;
R₆ est : H, un groupe alkyle en C₁-C₆, CO₂R₁₂, -C(O)NR₁₃R₁₄, naphtyle ou phényle éventuellement substitué par un ou plusieurs substituants choisis parmi : H, un groupe halogénoalkyle en C₁-C₆, alkyle en C₁-C₆, halogène, alcoxy en C₁-C₆, méthylènedioxy, nitro, cyano ;
R₇ est : H ou un groupe alkyle en C₁-C₆ ;
R₈, R₉, R₁₀ et R₁₁ sont indépendants et représentent : H, un groupe alkyle en C₁-C₆, un halogène, -OR₁₂, un groupe nitro, cyano, NR₁₃R₁₄ ; -COR₁₂ ; CO₂R₁₂ ; -SO₂NR₁₃R₁₄ ; -SO₂R₁₂ ; SR₁₂, -CONR₁₃R₁₄ ;
R₁₂ est H, un groupe alkyle en C₁-C₆ ou phényle ; et
R₁₃ et R₁₄ sont indépendants et représentent : H, un groupe alkyle en C₁-C₆ ou phényle ou R₁₃ et R₁₄, conjointement avec l'atome de N auquel ils sont attachés, forment un cycle à 5 ou 6 chaînons dans lequel il peut y avoir éventuellement un atome de N, O ou S ;
ou un sel, ou un solvate, ou un isomère géométrique, ou un isomère optique, ou un polymorphe de ceux-ci.

2. Composés selon la revendication 1, où Z est -CO- :
1-(benzo[b]thiophén-3-yl)-3-[4-(2-méthoxyphényl)-pipérazin-1-yl]propan-1-one
1-(3,5-diméthylbenzo[b]thiophén-2-yl)-3-[4-(2-méthoxyphényl)pipérazin-1-yl]propan-1-one

3. Composés selon la revendication 1, où Z est -CH(OR₆)- :
1-(benzo[b]thiophén-3-yl)-3-[4-(2-méthoxyphényl)-pipérazin-1-yl]propan-1-ol 1-(benzo[b]thiophén-3-yl)-3-[4-(2-hydroxyphényl)-pipérazin-1-yl]propan-1-ol 1-(3,5-diméthlbenzo[b]thiophén-2-yl)-3-[4-(2-méthoxyphényl)pipérazin-1-yl]propan-1-ol 1-(3-méthylbenzo[b]thiophén-2-yl)-3-[4-(2-méthoxyphényl)pipérazin-1-yl]propan-1-ol

4. Composés selon la revendication 1, où Z est -C(NOR₇)- :
1-(benzo[b]thiophén-3-yl)-3-[4-(2-méthoxyphényl)-pipérazin-1-yl]propan-1-one oxime

5. Utilisation d'un composé selon l'une quelconque des revendications 1 à 4 pour la préparation d'une composition pharmaceutique destinée au traitement de troubles neurologiques.

6. Utilisation selon la revendication 5 pour la préparation d'une composition pharmaceutique destinée au traitement de l'anxiété et/ou de la dépression.

7. Composition pharmaceutique comprenant un composé selon l'une quelconque des revendications 1 à 4, en une quantité thérapeutiquement active pour le traitement de l'anxiété et/ou de la dépression et une quantité adéquate d'un support.
